# EUROPEAN PATENT SPECIFICATION

(11) **EP 2 970 184 B1**
(45) Date of publication and mention of the grant of the patent: **05.12.2018**
(21) Application number: 14768759.4
(22) Date of filing: 10.03.2014
(51) Int. Cl.: C07D 401/04, C07D 403/04, A61K 31/4439, A61K 31/4155

(54) **COMPOUNDS AND USES THEREOF FOR THE MODULATION OF HEMOGLOBIN**
VERBINDUNGEN UND VERWENDUNGEN DAVON ZUR MODULATION VON HÄMOGLOBIN
COMPOSÉS ET LEURS UTILISATIONS POUR LA MODULATION D'HÉMOGLOBINE

(30) Priority: 15.03.2013 US 201313815810; 26.08.2013 US 201314010455
(43) Date of publication of application: 20.01.2016
(73) Proprietor: Global Blood Therapeutics, Inc., South San Francisco, CA 94080 (US)
(72) Inventor: HARRIS, Jason R., South San Francisco, California 94080 (US); METCALF, Brian W., South San Francisco, California 94080 (US); LI, Zhe, South San Francisco, California 94080 (US); GWALTNEY II, Stephen L., South San Francisco, California 94080 (US)
(74) Representative: Carpmaels & Ransford LLP
(86) International application number: PCT/US2014/022742
(87) International publication number: WO 2014/150261

(56) References cited:
- WO-A1-2008/116620
- WO-A1-2010/031589
- US-A1- 2005 096 337
- US-B2- 7 160 910

## Description

### FIELD OF THE INVENTION

This invention provides compounds and pharmaceutical compositions suitable for use as allosteric modulators of hemoglobin, methods and intermediates for their preparation, and methods for their use in treating disorders mediated by hemoglobin and disorders that would benefit from tissue and/or cellular oxygenation.

### STATE OF THE ART

Sickle cell disease is a disorder of the red blood cells, found particularly among those of African and Mediterranean descent. The basis for sickle cell disease is found in sickle hemoglobin (HbS), which contains a point mutation relative to the prevalent peptide sequence of hemoglobin (Hb).

Hemoglobin (Hb) transports oxygen molecules from the lungs to various tissues and organs throughout the body. Hemoglobin binds and releases oxygen through conformational changes. Sickle hemoglobin (HbS) contains a point mutation where glutamic acid is replaced with valine, allowing HbS to become susceptible to polymerization to give the HbS containing red blood cells their characteristic sickle shape. The sickled cells are also more rigid than normal red blood cells, and their lack of flexibility can lead to blockage of blood vessels. U.S. Patent No. 7, 160,910 discloses compounds that are allosteric modulators of hemoglobin. However, a need exists for additional therapeutics that can treat disorders that are mediated by Hb or by abnormal Hb such as HbS.

WO 2010/031589 A1 relates to small molecules reported to be bradykinin B2 receptor modulators.

### SUMMARY OF THE INVENTION

This invention relates generally to compounds and pharmaceutical compositions suitable as allosteric modulators of hemoglobin. In some aspects, this invention relates to methods for treating disorders mediated by hemoglobin and disorders that would benefit from tissue and/or cellular oxygenation.

The scope of the invention is defined by the appended claims. Any examples described herein not falling within the scope of the claims are described for reference purposes only.

The present invention provides a compound of formula (II): 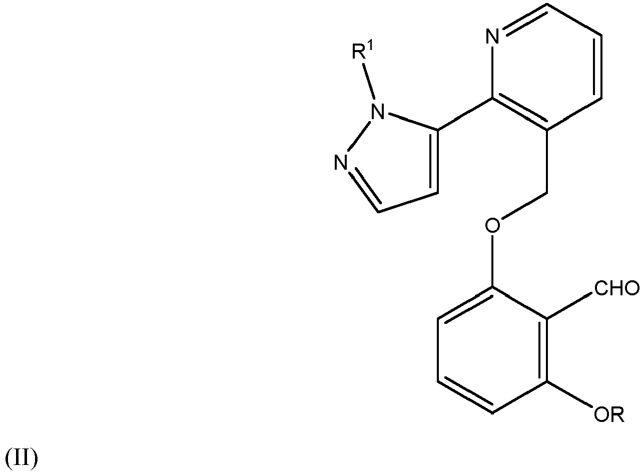 or an N oxide thereof, or a pharmaceutically acceptable salt of each thereof,
wherein R is a phosphate, a diphosphate, a phosphonate or a phosphoramidate containing moiety; or,
R is -C(O)R³¹, C(O)OR³¹, or CONR¹³R¹⁴, wherein each R³¹ is independently a C₁-C₆ alkyl, C₃-C₈ cycloalkyl, 4-9 membered heterocycle, or a 5-10 membered heteroaryl, containing at least 1 basic nitrogen moiety; and R¹³ and R¹⁴ independently are C₁-C₆ alkyl, C₃-C₈ cycloalkyl, 4-9 membered heterocycle, or a 5-10 membered heteroaryl, containing at least 1 basic nitrogen moiety; or R¹³ and R¹⁴ together with the nitrogen atom they are bonded to form a 4-9 membered heterocycle substituted with at least 1 amino, C₁-C₆ alkyl amino, or di C₁-C₆ alkylamino group; or
R is C(O)(CH₂)ₘNR³⁴R³⁵ or C(O)O(CH₂)ₘNR³⁴R³⁵, wherein m, R³⁴, and R³⁵ are defined as tabulated below:

| R | m | R³⁴ | R³⁵ | NR³⁴R³⁵ |
|---|---|---|---|---|
| C(O)(CH₂)ₘNR³⁴R³⁵ | 1 | Me | Me | |
| C(O)(CH₂)ₘNR³⁴R³⁵ | 2 | Me | Me | |
| C(O)(CH₂)ₘNR³⁴R³⁵ | 3 | Me | Me | |
| C(O)(CH₂)ₘNR³⁴R³⁵ | 4 | Me | Me | |
| C(O)(CH₂)ₘNR³⁴R³⁵ | 1 | | | 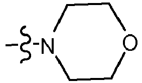 |
| C(O)(CH₂)ₘNR³⁴R³⁵ | 2 | | | 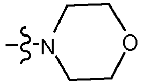 |
| C(O)(CH₂)ₘNR³⁴R³⁵ | 3 | | | 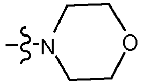 |
| C(O)(CH₂)ₘNR³⁴R³⁵ | 4 | | | 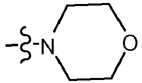 |
| C(O)O(CH₂)ₘNR³⁴R³⁵ | 2 | Me | Me | |
| C(O)O(CH₂)ₘNR³⁴R³⁵ | 3 | Me | Me | |
| C(O)O(CH₂)ₘNR³⁴R³⁵ | 4 | Me | Me | |
| C(O)O(CH₂)ₘNR³⁴R³⁵ | 2 | | | 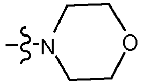 |
| C(O)O(CH₂)ₘNR³⁴R³⁵ | 3 | | | 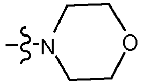 |
| C(O)O(CH₂)ₘNR³⁴R³⁵ | 4 | | | 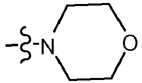 |

and R¹ is C₁-C₆ alkyl optionally substituted with 3-6 fluoro atoms.

Described is a compound of formula (I): 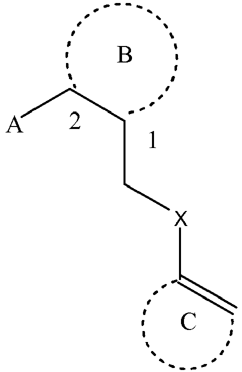 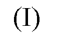 or an N oxide thereof, or a pharmaceutically acceptable salt of each thereof, wherein A is selected from the group consisting of: 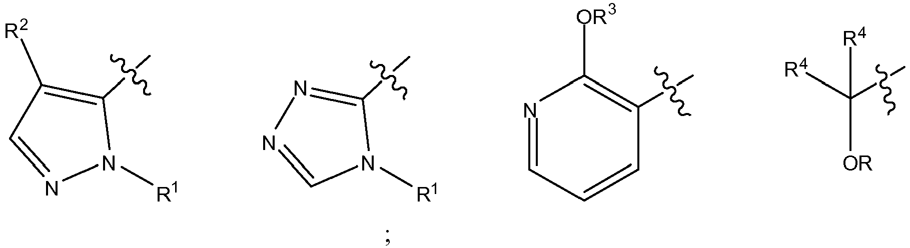 wherein R¹ is C₁-C₆ alkyl optionally substituted with 3-6 fluoro atoms;
R² is hydrogen or C₁-C₆ alkyl;
R³ is C₁-C₆ alkyl;
each R⁴ independently is hydrogen or C₁-C₆ alkyl;
ring B is 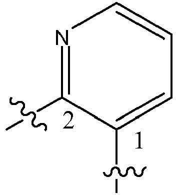 or ring B together with A is: 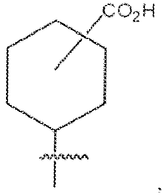 and stereoisomers thereof;
X is oxygen, S, SO, or SO₂;
ring C is selected from the group consisting of: 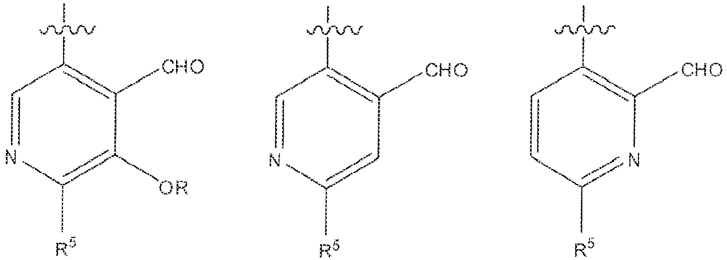 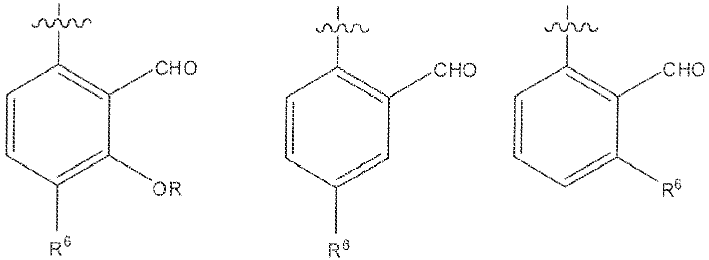 wherein R⁵ is selected from the group consisting of hydrogen; C₁-C₆ alkoxy, optionally substituted with a C₁-C₆ alkoxy group or with up to 3 fluoro atoms; C₁-C₆ alkyl; and halo;
R⁶ is hydrogen or halo;
R is hydrogen, a phosphate, a diphosphate, a phosphonate or a phosphoramidate containing moiety, or another promoiety ;
provided that the compound of formula (I) comprises at least 1 OR group where R is not hydrogen.

In another aspect, this invention provides a compound of formula (II): 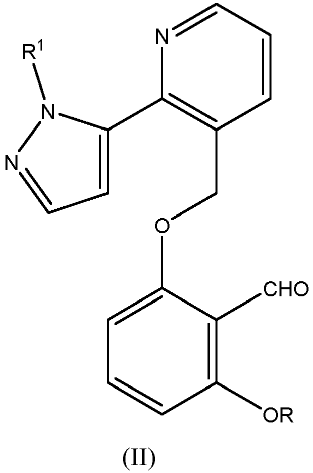 wherein
R is hydrogen, a phosphate or a diphosphate containing moiety, or another promoiety; and
R¹ is C₁-C₆ alkyl optionally substituted with 3-6 fluoro atoms;
provided that the compound of formula (I) comprises at least 1 OR group where R is not hydrogen.

In further aspects, this invention provides a compound of formula (II),
wherein R is -COR³¹, C(O)OR³¹, or CONR¹³R¹⁴,
each R³¹ is independently a C₁-C₆ alkyl; C₃-C₈ cycloalkyl, 4-9 membered heterocycle, or a 5-10 membered heteroaryl, containing at least 1 basic nitrogen moiety; and
R¹³ and R¹⁴ independently are C₁-C₆ alkyl; C₃-C₈ cycloalkyl, 4-9 membered heterocycle, or a 5-10 membered heteroaryl, containing at least 1 basic nitrogen moiety; or R¹³ and R¹⁴ together with the nitrogen atom they are bonded to for a 4-9 member heterocycle substituted with at least 1 amino, C₁-C₆ alkyl amino, or di C₁-C₆ alkylamino group.

In one embodiment, this invention provides a compound of formula (II), wherein R¹ is isopropyl.

In certain aspects, this invention provides a compound of formula (II): 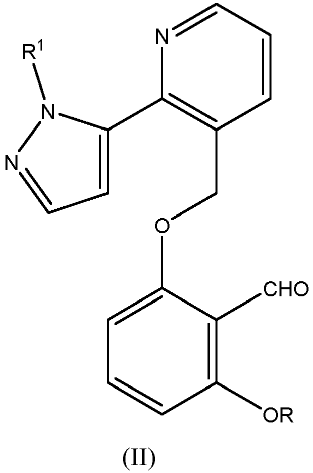 wherein R is phosphate, C(O)(CH₂)ₘNR³⁴R³⁵, or C(O)O(CH₂)ₘNR³⁴R³⁵; and
wherein m, R³⁴ and R³⁵ are defined as tabulated below:

| R | m | R³⁴ | R³⁵ | NR³⁴R³⁵ |
|---|---|---|---|---|
| C(O)(CH₂)ₘNR³⁴R³⁵ | 1 | Me | Me | |
| C(O)(CH₂)ₘNR³⁴R³⁵ | 2 | Me | Me | |
| C(O)(CH₂)ₘNR³⁴R³⁵ | 3 | Me | Me | |
| C(O)(CH₂)ₘNR³⁴R³⁵ | 4 | Me | Me | |
| C(O)(CH₂)ₘNR³⁴R³⁵ | 1 | | | 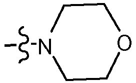 |
| C(O)(CH₂)ₘNR³⁴R³⁵ | 2 | | | 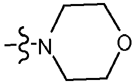 |
| C(O)(CH₂)ₘNR³⁴R³⁵ | 3 | | | 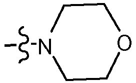 |
| C(O)(CH₂)ₘNR³⁴R³⁵ | 4 | | | 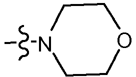 |
| C(O)O(CH₂)ₘNR³⁴R³⁵ | 2 | Me | Me | |
| C(O)O(CH₂)ₘNR³⁴R³⁵ | 3 | Me | Me | |
| C(O)O(CH₂)ₘNR³⁴R³⁵ | 4 | Me | Me | |
| C(O)O(CH₂)ₘNR³⁴R³⁵ | 2 | | | 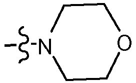 |
| C(O)O(CH₂)ₘNR³⁴R³⁵ | 3 | | | 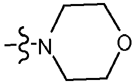 |
| C(O)O(CH₂)ₘNR³⁴R³⁵ | 4 | | | 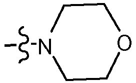 |

an N oxide thereof, or a pharmaceutically acceptable salt of each thereof.

This invention arises in part out of the discovery that a phosphate prodrug of a compound of formula (III): 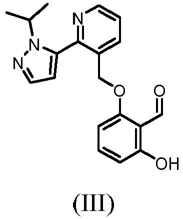 provides enhanced aqueous solubility while providing a whole blood exposure equivalent to that of the compound of formula (III) upon oral administration. This invention relates generally to compounds and pharmaceutical compositions suitable for use as allosteric modulators of hemoglobin. In some aspects, this invention relates to methods for treating disorders mediated by hemoglobin and disorders that would benefit from tissue and/or cellular oxygenation.

In a further aspect of the invention, a compound of formula (IV) is provided: 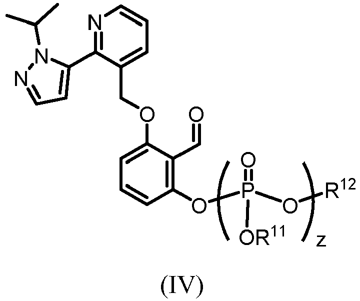 or a salt, or a pharmaceutically acceptable salt thereof.

In further aspects of the invention, a pharmaceutical composition is provided where the pharmaceutical composition comprises any of the compounds disclosed herein, and at least one pharmaceutically acceptable excipient.

In further aspects of the invention, compounds or pharmaceutical compositions of the invention are provided for use in a method for treating a condition associated with oxygen deficiency.

In further aspects of the invention, compounds or pharmaceutical compositions of the invention are provided for use in a method for treating cancer, a pulmonary disorder, stroke, high altitude sickness, an ulcer, a pressure sore, Alzheimer's disease, acute respiratory disease syndrome, or a wound.

In further aspects of the invention, compounds or pharmaceutical compositions of the invention are provided for use in a method for treating sickle cell disease.

Described is a method is provided for increasing oxygen affinity of hemoglobin S in a subject, the method comprising administering to a subject in need thereof a therapeutically effective amount of any of the compounds or compositions disclosed herein.

In further aspects of the invention, compounds or pharmaceutical compositions of the invention are provided for use in a method for treating oxygen deficiency associated with sickle cell anemia.
Methods for increasing oxygen affinity of hemoglobin S and methods for treating oxygen deficiency associated with sickle cell anemia are well known and/or will be apparent to the skilled artisan in view of this disclosure.

### BRIEF DESCRIPTION OF THE FIGURE

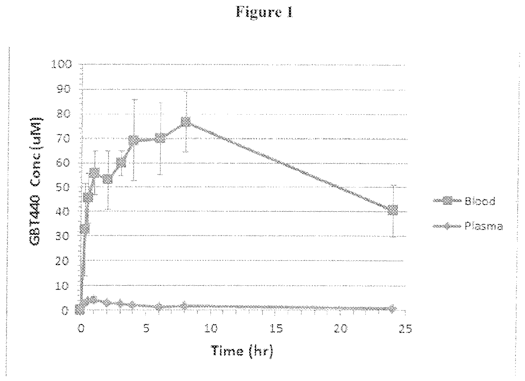

Figure 1 graphically illustrates the *in vivo* release of the compound of formula (III) from a monophosphate prodrug compound (V-B) of this invention, when administered at 10 mg/kg to a test subject.

### DETAILED DESCRIPTION OF THE INVENTION

It must be noted that as used herein and in the appended claims, the singular forms "a", "an", and "the" include plural referents unless the context clearly dictates otherwise. Thus, for example, reference to "a solvent" includes a plurality of such solvents.

As used herein, the term "comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition or process consisting essentially of the elements as defined herein would not exclude other materials or steps that do not materially affect the basic and novel characteristic(s) of the claimed invention. "Consisting of" shall mean excluding more than trace elements of other ingredients and substantial method steps. Embodiments defined by each of these transition terms are within the scope of this invention.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations. Each numerical parameter should at least be construed in light of the number of reported significant digits and by applying ordinary rounding techniques. The term "about" when used before a numerical designation, *e.g*., temperature, time, amount, and concentration, including range, indicates approximations which may vary by ( +) or (-) 10 %, 5 % or 1 %.

As used herein, Cₘ-Cₙ, such as C₁-C₁₂, C₁-C₈, or C₁-C₆ when used before a group refers to that group containing m to n carbon atoms.

The term "alkoxy" refers to -O-alkyl.

The term "alkyl" refers to monovalent saturated aliphatic hydrocarbyl groups having from 1 to 12 carbon atoms (*i.e.,* C₁-C₁₂ alkyl) or 1 to 8 carbon atoms (*i.e.,* C₁-C₈ alkyl). or 1 to 4 carbon atoms. This term includes, by way of example, linear and branched hydrocarbyl groups such as methyl (CH₃-), ethyl (CH₃CH₂-), *n*-propyl (CH₃CH₂CH₂-), isopropyl ((CH₃)₂CH-), *n*-butyl (CH₃CH₂CH₂CH₂-), isobutyl ((CH₃)₂CHCH₂-), *sec*-butyl ((CH₃)(CH₃CH₂)CH-), *t*-butyl ((CH₃)₃C-), n-pentyl (CH₃CH₂CH₂CH₂CH₂-), and neopentyl ((CH₃)₃CCH₂-).

The term "aryl" refers to a monovalent, aromatic mono- or bicyclic ring having 6-10 ring carbon atoms. Examples of aryl include phenyl and naphthyl. The condensed ring may or may not be aromatic provided that the point of attachment is at an aromatic carbon atom. For example, and without limitation, the following is an aryl group:

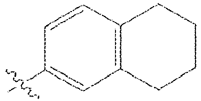

The term "-CO₂H ester" refers to an ester formed between the -CO₂H group and an alcohol, preferably an aliphatic alcohol. A preferred example included -CO₂R¹³, wherein R¹³ is alkyl or aryl group optionally substituted with an amino group.

The term "chiral moiety" refers to a moiety that is chiral. Such a moiety can possess one or more asymmetric centers. Preferably, the chiral moiety is enantiomerically enriched, and more preferably a single enantiomer. Non limiting examples of chiral moieties include chiral carboxylic acids, chiral amines, chiral amino acids, such as the naturally occurring amino acids, chiral alcohols including chiral steroids, and the likes.

The term "cycloalkyl" refers to a monovalent, preferably saturated, hydrocarbyl mono-, bi-, or tricyclic ring having 3-12 ring carbon atoms. While cycloalkyl, refers preferably to saturated hydrocarbyl rings, as used herein, it also includes rings containing 1-2 carbon-carbon double bonds. Nonlimiting examples of cycloalkyl include cyclopropyl, cyclobutyl, cyclopentyl, cyclohexyl, cycloheptyl, adamentyl, and the like. The condensed rings may or may not be non-aromatic hydrocarbyl rings provided that the point of attachment is at a cycloalkyl carbon atom. For example, and without limitation, the following is a cycloalkyl group:

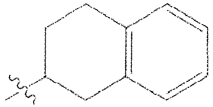

The term "halo" refers to F, Cl, Br, and/or I.

The term "heteroaryl" refers to a monovalent, aromatic mono-, bi-, or tricyclic ring having 2-16 ring carbon atoms and 1-8 ring heteroatoms selected preferably from N, O, S, and P and oxidized forms of N, S, and P, provided that the ring contains at least 5 ring atoms. Nonlimiting examples of heteroaryl include furan, imidazole, oxadiazole, oxazole, pyridine, quinoline, and the like. The condensed rings may or may not be a heteroatom containing aromatic ring provided that the point of attachment is a heteroaryl atom. For example, and without limitation, the following is a heteroaryl group:

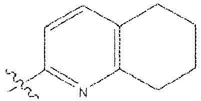

The term "heterocyclyl" or hetcrocycle refers to a non-aromatic, mono-, bi-, or tricyclic ring containing 2-12 ring carbon atoms and 1-8 ring heteroatoms selected preferably from N, O, S, and P and oxidized forms of N, S, and P, provided that the ring contains at least 3 ring atoms. While heterocyclyl preferably refers to saturated ring systems, it also includes ring systems containing 1-3 double bonds, provided that they ring is non-aromatic. Nonlimiting examples of heterocyclyl include, azalactones, oxazoline, piperidinyl, piperazinyl, pyrrolidinyl, tetrahydrofuranyl, and tetrahydropyranyl. The condensed rings may or may not contain a non-aromatic heteroatom containing ring provided that the point of attachment is a heterocyclyl group. For example, and without limitation, the following is a heterocyclyl group:

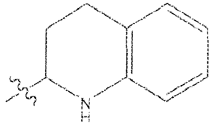

The term "hydrolyzing" refers to breaking an R^{H}-O-CO-, R^{H}-O-CS-, or an R^{H}-O-SO₂- moiety to an R^{H}-OH, preferably by adding water across the broken bond. A hydrolyzing is performed using various methods well known to the skilled artisan, non limiting examples of which include acidic and basic hydrolysis.

The term "oxo" refers to a C=O group, and to a substitution of 2 geminal hydrogen atoms with a C=O group.

The term "optionally substituted" refers to a substituted or unsubstituted group. The group may be substituted with one or more substituents, such as e.g., 1, 2. 3. 4 or 5 substituents. Preferably, the substituents are selected from the group consisting of oxo, halo, -CN, NO₂, -N₂+, -CO₂R¹⁰⁰, -OR¹⁰⁰, -SR¹⁰⁰, -SOR¹⁰⁰, -SO₂R¹⁰⁰, -NR¹⁰¹R¹⁰², -CONR¹⁰¹R¹⁰²,-SO₂NR¹⁰¹R¹⁰², C₁-C₆ alkyl, C₁-C₆ alkoxy, -CR¹⁰⁰=C(R¹⁰⁰)₂, -CCR¹⁰⁰, C₃-C₁₀ cycloalkyl, C₃-C₁₀ heterocyclyl, C₆-C₁₂ aryl and C₂-C₁₂ heteroaryl, wherein each R¹⁰⁰ independently is hydrogen or C₁-C₈ alkyl; C₃-C₁₂ cycloalkyl; C₃-C₁₀ heterocyclyl; C₆-C₁₂ aryl; or C₂-C₁₂ heteroaryl; wherein each alkyl, cycloalkyl, heterocyclyl, aryl, or heteroaryl is optionally substituted with 1-3 halo, 1-3 C₁-C₆ alkyl, 1-3 C₁-C₆ haloalkyl or 1-3 C₁-C₆ alkoxy groups. Preferably, the substituents are selected from the group consisting of chloro, fluoro, -OCH₃, methyl, ethyl, *iso*-propyl, cyclopropyl, vinyl, ethynyl, -CO₂H, -CO₂CH₃, -OCF₃, -CF₃ and-OCHF₂.

As used herein, R¹⁰¹ and R¹⁰² independently is hydrogen; C₁-C₈ alkyl, optionally substituted with -CO₂H or an ester thereof, C₁-C₆ alkoxy, oxo, -CR¹⁰³=C(R¹⁰³)₂, -CCR, C₃-C₁₀ cycloalkyl, C₃-C₁₀ heterocyclyl, C₆-C₁₂ aryl, or C₂-C₁₂ heteroaryl, wherein each R¹⁰³ independently is hydrogen or C₁-C₈ alkyl; C₃-C₁₂ cycloalkyl; C₃-C₁₀ heterocyclyl; C₆-C₁₂ aryl; or C₂-C₁₂ heteroaryl; wherein each cycloalkyl, heterocyclyl, aryl, or heteroaryl is optionally substituted with 1-3 alkyl groups or 1-3 halo groups, or R¹⁰¹ and R¹⁰² together with the nitrogen atom they are attached to form a 5-7 membered heterocycle.

The term "protecting group" as used herein, is well known in the art and includes those described in detail in Protecting Groups in Organic Synthesis, T. W. Greene and P. G. M. Wuts, 3rd edition, John Wiley & Sons, 1999, and subsequent revisions, the entirety of each of which is incorporated herein by reference. Suitable protecting groups include benzyl. methyl, methoxylmethyl (MOM), methylthiomethyl (MTM), *t*-butylthiomethyl. (phenyldimethylsilyl)methoxymethyl (SMOM), benzyloxymethyl (BOM), *p-*methoxybenzyloxymethyl (PMBM), (4-methoxyphenoxy)methyl (*p*-AOM), guaiacolmethyl (GUM), *t*-butoxymethyl, 4-pentenyloxymethyl (POM), siloxymethyl, 2-methoxyethoxymethyl (MEM), 2,2,2-trichloroethoxymethyl, bis(2-chloroethoxy)methyl, 2-(trimethylsilyl)ethoxymethyl (SEMOR), tetrahydropyranyl (THP), 3-bromotetrahydropyranyl, tetrahydrothiopyranyl, 1-methoxycyclohexyl, 4-methoxytetrahydropyranyl (MTHP), 4-methoxytetrahydrothiopyranyl, 4-methoxytetrahydrothiopyranyl S,S-dioxide, 1-[(2-chloro-4-methyl)phenyl]-4-methoxypiperidin-4-yl (CTMP), 1,4-dioxan-2-yl, tetrahydrofuranyl, tetrahydrothiofuranyl, 2,3,3a,4,5,6,7,7a-octahydro-7,8,8-trimethyl-4,7-methanobenzofuran-2-yl, 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, *t*-butyl, allyl, *p*-chlorophenyl, *p*-methoxyphenyl, 2,4-dinitrophenyl, benzyl, *p*-methoxybenzyl, 3,4-dimethoxybenzyl, *o*-nitrobenzyl, *p*-nitrobenzyl, *p-*halobenzyl, 2,6-dichlorobenzyl, *p*-cyanobenzyl, *p*-phenylbenzyl, 2-picolyl, 4-picolyl, 3-methyl-2-picolyl *N*-oxido, diphenylmethyl, *p,p*'-dinitrobenzhydryl, 5-dibenzosuberyl, triphenylmethyl, α-naphthyldiphenylmethyl, *p*-methoxyphenyldiphenylmethyl. di(*p-*methoxyphenyl)phenylmethyl, tri(*p*-methoxyphenyl)methyl, 4-(4'-bromophenacyloxyphenyl)diphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 3-(imidazol-1-yl)bis(4',4"-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodithiolan-2-yl, benzisothiazolyl S,S-dioxido, trimethylsilyl (TMS), triethylsilyl (TES), triisopropylsilyl (TIPS), dimethylisopropylsilyl (IPDMS), diethylisopropylsilyl (DEIPS), dimethylthexylsilyl, *t*-butyldimethylsilyl (TBDMS). *t-*butyldiphenylsilyl (TBDPS), tribenzylsilyl, tri-*p*-xylylsilyl, triphenylsilyl, diphenylmethylsilyl (DPMS), *t*-butylmethoxyphenylsilyl (TBMPS), formate, benzoylformate, acetate, chloroacetate, dichloroacetate, trichloroacetate, trifluoroacetate, methoxyacetate, triphenylmethoxyacetate, phenoxyacetate, *p*-chlorophenoxyacetate, 3-phenylpropionate, 4-oxopentanoate (levulinate), 4,4-(ethylenedithio)pentanoate (levulinoyldithioacetal), pivaloate, adamantoate, crotonate, 4-methoxycrotonate, benzoate, *p-*phenylbenzoate, 2,4,6-trimethylbenzoate (mesitoate), alkyl methyl carbonate, 9-fluorenylmethyl carbonate (Fmoc), alkyl ethyl carbonate, alkyl 2,2,2-trichloroethyl carbonate (Troc), 2-(trimethylsilyl)ethyl carbonate (TMSEC), 2-(phenylsulfonyl) ethyl carbonate (Psec). 2-(triphenylphosphonio) ethyl carbonate (Peoc), alkyl isobutyl carbonate, alkyl vinyl carbonate alkyl allyl carbonate, alkyl *p*-nitrophenyl carbonate, alkyl benzyl carbonate, alkyl *p*-methoxybenzyl carbonate, alkyl 3,4-dimethoxybenzyl carbonate, alkyl *o*-nitrobenzyl carbonate, alkyl *p*-nitrobenzyl carbonate, alkyl *S*-benzyl thiocarbonate, 4-ethoxy-1-napththyl carbonate, methyl dithiocarbonate, 2-iodobenzoate, 4-azidobutyrate. 4-nitro-4-methylpentanoate. *o*-(dibromomethyl)benzoate, 2-formylbenzenesulfonate. 2-(methylthiomethoxy)ethyl, 4-(methylthiomethoxy)butyrate, 2-(methylthiomethoxymethyl)benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (*E*)-2-methyl-2-butenoate, *o-*(methoxycarbonyl)benzoate, α-naphthoate, nitrate, alkyl *N,N.N',N'-*tetramethylphosphorodiamidate, alkyl *N*-phenylcurbamate. borate, dimethylphosphinothioyl, alkyl 2,4-dinitrophenylsulfonate, sulfate, methanesulfonate (mesylate), benzylsulfonate, and tosylate (Ts). For protecting 1,2- or 1,3-diols, the protecting groups include methylene acetal, ethylidene acetal, 1-*t*-butylethylidene ketal, 1-phenylethylidene ketal, (4-methoxyphenyl)ethylidene acetal, 2,2,2-trichloroethylidene acetal, acetonide, cyclopentylidene ketal, cyclohexylidene ketal, cycloheptylidene ketal, benzylidene acetal, *p-*methoxybenzylidene acetal, 2,4-dimethoxybenzylidene ketal, 3,4-dimethoxybenzylidenc acetal, 2-nitrobenzylidene acetal, methoxymethylene acetal, ethoxymethylene acetal, dimethoxymethylene ortho ester, 1-methoxyethylidene ortho ester, 1-ethoxyethylidine ortho ester, 1,2-dimethoxyethylidene ortho ester, α-methoxybenzylidene ortho ester, 1-(*N,N-*dimethylamino)ethylidene derivative, α-(*N,N*'-dimethylamino)benzylidene derivative, 2-oxacyclopentylidene ortho ester, di-*t-*butylsilylene group (DTBS), 1,3-(1,1,3,3-tetraisopropyldisiloxanylidene) derivative (TIPDS), tetra-*t*-butoxydisiloxane-1,3-diylidene derivative (TBDS), cyclic carbonates, cyclic boronates, ethyl boronate, and phenyl boronate.

The term "pharmaceutically acceptable" refers to safe and non-toxic for *in vivo,* preferably, human administration.

The term "pharmaceutically acceptable salt" refers to a salt that is pharmaceutically acceptable.

The term "salt" refers to an ionic compound formed between an acid and a base. When the compound provided herein contains an acidic functionality, such salts include, without limitation, alkali metal, alkaline earth metal, and ammonium salts. As used herein, ammonium salts include, salts containing protonated nitrogen bases and alkylated nitrogen bases. Exemplary, and non-limiting cations useful in pharmaceutically acceptable salts include Na, K, Rb, Cs, NH₄, Ca, Ba, imidazolium, and ammonium cations based on naturally occurring amino acids. When the compounds utilized herein contain basic functionally, such salts include, without limitation, salts of organic acids, such as carboxylic acids and sulfonic acids, and mineral acids, such as hydrogen halides, sulfuric acid, phosphoric acid, and the likes. Exemplary and non-limiting anions useful in pharmaceutically acceptable salts include oxalate, maleate, acetate, propionate, succinate. tartrate, chloride, sulfate. bisalfate, mono-di-, and tribasic phosphate, mesylate, tosylate, and the likes.

The terms "treat", "treating" or "treatment", as used herein, include alleviating, abating or ameliorating a disease or condition or one or more symptoms thereof, preventing additional symptoms, ameliorating or preventing the underlying metabolic causes of symptoms, inhibiting the disease or condition, *e.g*., arresting or suppressing the development of the disease or condition, relieving the disease or condition, causing regression of the disease or condition, relieving a condition caused by the disease or condition, or suppressing the symptoms of the disease or condition, and are intended to include prophylaxis. The terms also include relieving the disease or conditions, *e.g*., causing the regression of clinical symptoms. The terms further include achieving a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the individual, notwithstanding that the individual is still be afflicted with the underlying disorder. For prophylactic benefit, the compositions are administered to an individual at risk of developing a particular disease, or to an individual reporting one or more of the physiological symptoms of a disease, even though a diagnosis of this disease has not been made.

The terms "preventing" or "prevention" refer to a reduction in risk of acquiring a disease or disorder (*i.e.,* causing at least one of the clinical symptoms of the disease not to develop in a subject that may be exposed to or predisposed to the disease but does not yet experience or display symptoms of the disease). The terms further include causing the clinical symptoms not to develop, for example in a subject at risk of suffering from such a disease or disorder, thereby substantially averting onset of the disease or disorder.

The term "effective amount" refers to an amount that is effective for the treatment of a condition or disorder by an intranasal administration of a compound or composition described herein. In some embodiments, an effective amount of any of the compositions or dosage forms described herein is the amount used to treat a disorder mediated by hemoglobin or a disorder that would benefit from tissue and/or cellular oxygenation of any of the compositions or dosage forms described herein to a subject in need thereof.

The term "carrier" as used herein, refers to relatively nontoxic chemical compounds or agents that facilitate the incorporation of a compound into cells, *e.g.,* red blood cells. or tissues.

As used herein, a "prodrug" is a compound that, after administration, is metabolized or otherwise converted to an active or more active form with respect to at least one property. To produce a prodrug, a pharmaceutically active compound can be modified chemically to render it less active or inactive, but the chemical modification is such that an active form of the compound is generated by metabolic or other biological processes. A prodrug may have, relative to the drug, altered metabolic stability or transport characteristics, fewer side effects or lower toxicity. For example, see the reference Nogrady, 1985, Medicinal Chemistry A Biochemical Approach, Oxford University Press, New York, pages 388-392. Prodrugs can also be prepared using compounds that are not drugs.

The invention provides prodrugs of substituted benzaldehyde compounds that increase oxygen affinity of hemoglobin S. The structures of the compounds, and derivatives thereof, as well as methods of their synthesis, pharmaceutical formulations thereof and methods of use are also provided.

### Compounds

In certain aspects of the invention, a compound of formula (II) is provided: 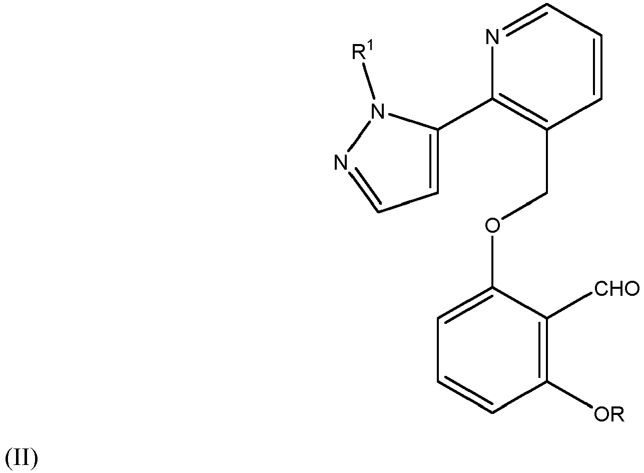 or an N oxide thereof, or a pharmaceutically acceptable salt of each thereof,
wherein R is a phosphate, a diphosphate, a phosphonate or a phosphoramidate containing moiety; or,
R is -C(O)R³¹, C(O)OR³¹, or CONR¹³R¹⁴, wherein
each R³¹ is independently a C₁-C₆ alkyl, C₃-C₈ cycloalkyl, 4-9 membered heterocycle, or a 5-10 membered heteroaryl, containing at least 1 basic nitrogen moiety; and R¹³ and R¹⁴ independently are C₁-C₆ alkyl, C₃-C₈ cycloalkyl, 4-9 membered heterocycle, or a 5-10 membered heteroaryl, containing at least 1 basic nitrogen moiety; or R¹³ and R¹⁴ together with the nitrogen atom they are bonded to form a 4-9 membered heterocycle substituted with at least 1 amino, C₁-C₆ alkyl amino, or di C₁-C₆ alkylamino group; or R is C(O)(CH₂)ₘNR³⁴R³⁵ or C(O)O(CH₂)ₘNR³⁴R³⁵,
wherein m, R³⁴, and R³⁵ are defined as tabulated below:

| R | m | R³⁴ | R³⁵ | NR³⁴R³⁵ |
|---|---|---|---|---|
| C(O)(CH₂)ₘNR³⁴R³⁵ | 1 | Me | Me | |
| C(O)(CH₂)ₘNR³⁴R³⁵ | 2 | Me | Me | |
| C(O)(CH₂)ₘNR³⁴R³⁵ | 3 | Me | Me | |
| C(O)(CH₂)ₘNR³⁴R³⁵ | 4 | Me | Me | |
| C(O)(CH₂)ₘNR³⁴R³⁵ | 1 | | | 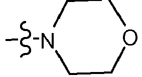 |
| C(O)(CH₂)ₘNR³⁴R³⁵ | 2 | | | 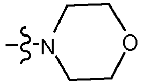 |
| C(O)(CH₂)ₘNR³⁴R³⁵ | 3 | | | 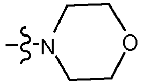 |
| C(O)(CH₂)ₘNR³⁴R³⁵ | 4 | | | 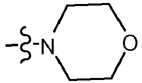 |
| C(O)O(CH₂)ₘNR³⁴R³⁵ | 2 | Me | Me | |
| C(O)O(CH₂)ₘNR³⁴R³⁵ | 3 | Me | Me | |
| C(O)O(CH₂)ₘNR³⁴R³⁵ | 4 | Me | Me | |
| C(O)O(CH₂)ₘNR³⁴R³⁵ | 2 | | | 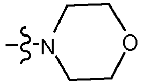 |
| C(O)O(CH₂)ₘNR³⁴R³⁵ | 3 | | | 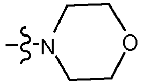 |
| C(O)O(CH₂)ₘNR³⁴R³⁵ | 4 | | | 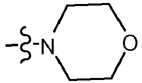 |
| P(O)(OH)₂ | | | | |

and R¹ is C₁-C₆ alkyl optionally substituted with 3-6 fluoro atoms

Described is a compound of formula (I): 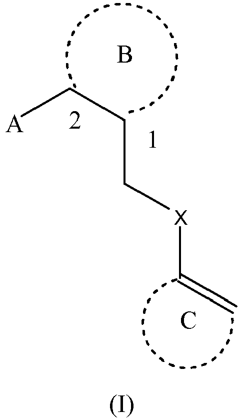 or an N oxide thereof, or a pharmaceutically acceptable salt of each thereof, wherein
A is selected from the group consisting of: 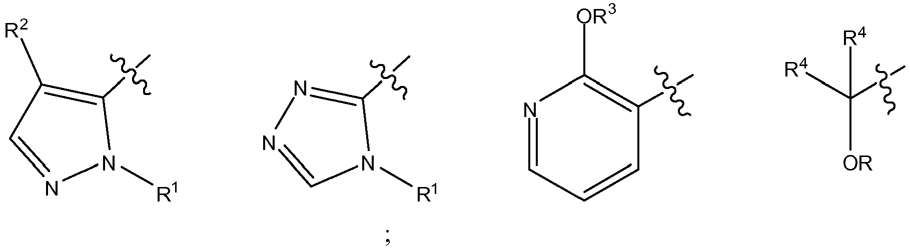 wherein R¹ is C₁-C₆ alkyl optionally substituted with 3-6 fluoro atoms;
R² is hydrogen or C₁-C₆ alkyl;
R³ is C₁-C₆ alkyl;
each R⁴ independently is hydrogen or C₁-C₆ alkyl;
ring B is 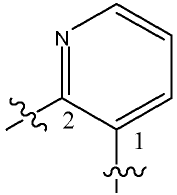 or ring B together with A is: 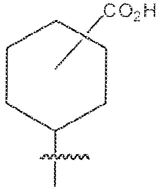 X is oxygen, S, SO, or SO₂;
ring C is selected from the group consisting of: 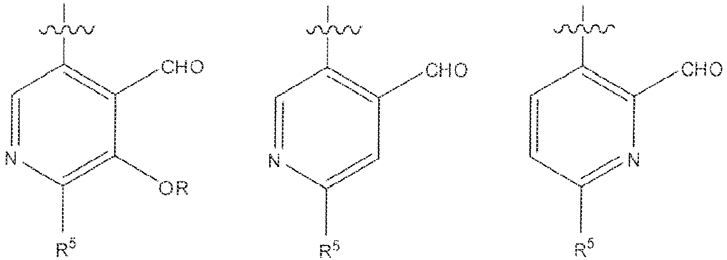 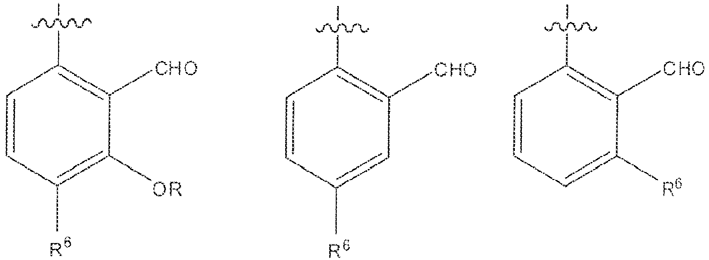 wherein R⁵ is selected from the group consisting of hydrogen; C₁-C₆ alkoxy, optionally substituted with a C₁-C₆ alkoxy group or with up to 3 fluoro atoms; C₁-C₆ alkyl; and halo;
R⁶ is hydrogen or halo;
R is hydrogen, a phosphate, a diphosphate, a phosphonate or a phosphoramidate containing moiety, or another promoiety;
provided that the compound of formula (I) comprises at least 1 OR group where R is not hydrogen; and
the promoieties are structurally and functionally defined herein.

In certain embodiments, a compound of formula (II) is provided: 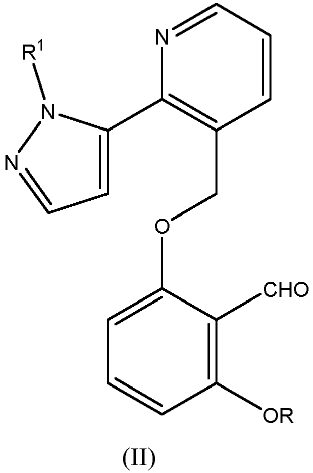 wherein
R is hydrogen, a phosphate, a diphosphate, a phosphonate or a phosphoramidate containing moiety, or another promoiety;
R¹ is C₁-C₆ alkyl optionally substituted with 3-6 fluoro atoms;
provided that the compound of formula (I) comprises at least 1 OR group where R is not hydrogen and
the promoieties are structurally and functionally defined herein.

In one aspect, **R** is a phosphate, a diphosphate, a phosphonate or a phosphoramidate containing moiety. Preferably the prodrug moiety imparts at least a 2 fold, more preferably a 4 fold, enhanced solubility and/or bioavailability to the active moiety (where R is hydrogen), and more preferably is hydrolyzed in vivo.

In certain embodiments, R is -C(O)R³¹, C(O)OR³¹, or CONR¹³R¹⁴,
each R³¹ is independently a C₁-C₆ alkyl; C₃-C₈ cycloalkyl, 4-9 membered heterocycle, or a 5-10 membered heteroaryl, containing at least 1 basic nitrogen moiety; and
R¹³ and R¹⁴ independently are C₁-C₆ alkyl; C₃-C₈ cycloalkyl, 4-9 membered heterocycle, or a 5-10 membered heteroaryl, containing at least 1 basic nitrogen moiety; or R¹³ and R¹⁴ together with the nitrogen atom they are bonded to for a 4-9 member heterocycle substituted with at least 1 amino, C₁-C₆ alkyl amino, or di C₁-C₆ alkylamino group.

Preferably, R¹ is isopropyl.

In a preferred embodiment, linkage of the prodrug moiety to the rest of the active molecule is stable enough so that the serum half life of the prodrug is from about 8 to about 24 hours.

In an embodiment of the invention, the prodrug moiety comprises a tertiary amine having a pKa near the physiological pH of 7.5. Any amines having a pKa within 1 unit of 7.5 are suitable alternatives amines for this purpose. The amine may be provided by the amine of a morpholino group. This pKa range of 6.5 to 8.5 allows for significant concentrations of the basic neutral amine to be present in the mildly alkaline small intestine. The basic, neutral form of the amine prodrug is lipophilic and is absorbed through the wall of the small intestine into the blood. Following absorption into the bloodstream, the prodrug moiety is cleaved by esterases which are naturally present in the serum to release an active compound.

Examples of **R** include, without limitation: 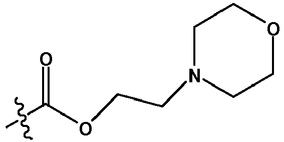 , and

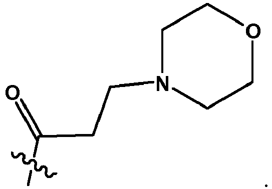

In another embodiment, R is as tabulated below:

| R | m | R³⁴ | R³⁵ | NR³⁴R³⁵ |
|---|---|---|---|---|
| C(O)(CH₂)ₘNR³⁴R³⁵ | 1 | Me | Me | |
| C(O)(CH₂)ₘNR³⁴R³⁵ | 2 | Me | Me | |
| C(O)(CH₂)ₘNR³⁴R³⁵ | 3 | Me | Me | |
| C(O)(CH₂)ₘNR³⁴R³⁵ | 4 | Me | Me | |
| C(O)(CH₂)ₘNR³⁴R³⁵ | 1 | | | 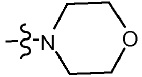 |
| C(O)(CH₂)ₘNR³⁴R³⁵ | 2 | | | 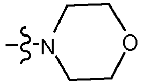 |
| C(O)(CH₂)ₘNR³⁴R³⁵ | 3 | | | 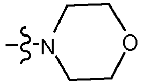 |
| C(O)(CH₂)ₘNR³⁴R³⁵ | 4 | | | 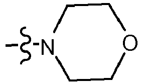 |
| C(O)O(CH₂)ₘNR³⁴R³⁵ | 2 | Me | Me | |
| C(O)O(CH₂)ₘNR³⁴R³⁵ | 3 | Me | Me | |
| C(O)O(CH₂)ₘNR³⁴R³⁵ | 4 | Me | Me | |
| C(O)O(CH₂)ₘNR³⁴R³⁵ | 2 | | | 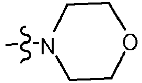 |
| C(O)O(CH₂)ₘNR³⁴R³⁵ | 3 | | | 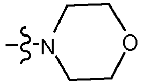 |
| C(O)O(CH₂)ₘNR³⁴R³⁵ | 4 | | | 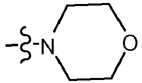 |
| P(O)(OH)₂ | | | | |

an N oxide thereof, or a pharmaceutically acceptable salt of each thereof.

Each heterocyclic and heteroaryl ring system is optionally substituted with C₁-C₃ alkyl, -OH, amino and carboxyl groups.

Each heterocyclic is optionally substituted with one or more, preferably, 1-3, C₁-C₃ alkyl, -OH, amino and/or carboxyl groups.

Each heterocyclic and heteroaryl ring system is optionally substituted with one or more, preferably 1-3, C₁-C₃ alkyl, -OH, amino and/or carboxyl groups.

In another aspect, **R** comprises a cleavable linker, wherein the term "cleavable linker" refers to a linker which has a short half life in vivo. The breakdown of the linker Z in a compound releases or generates the active compound. In one embodiment, the cleavable linker has a half life of less than ten hours. In one embodiment, the cleavable linker has a half life of less than an hour. In one embodiment, the half life of the cleavable linker is between one and fifteen minutes. In one embodiment, the cleavable linker has at least one connection with the structure: C*-C(=X*)X*-C* wherein C* is a substituted or unsubstituted methylene group, and X* is S or O. In one embodiment, the cleavable linker has at least one C*-C(=O)O-C* connection. In one embodiment, the cleavable linker has at least one C*-C(=O)S-C* connection. In one embodiment, the cleavable linker has at least one -C(=O)N*-C*-SO₂-N*-connection, wherein N* is -NH- or C₁-C₆ alkylamino. In one embodiment, the cleavable linker is hydrolyzed by an esterase enzyme.

In one embodiment, the linker is a self-immolating linker, such as that disclosed in U.S. patent publication 2002/0147138, to Firestone; PCT Appl. No. US05/08161 and PCT Pub. No. 2004/087075. In another embodiment, the linker is a substrate for enzymes. See generally Rooseboom et al., 2004, Pharmacol. Rev. 56:53-102.

In certain aspects of the invention, prodrugs of a compound of formula (III) are provided: 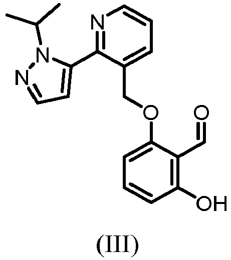 or a pharmaceutically acceptable salt thereof.

In further aspects of the invention, a compound of formula (IV) is provided: 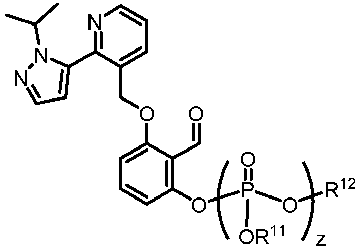 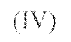 or a pharmaceutically acceptable salt thereof, wherein
R¹¹ and R¹² are independently selected from the group consisting of
hydrogen,
C₁-C₆ alkyl optionally substituted with 1-3 C₆-C₁₂ aryl groups, optionally substituted;
C₆-C₁₂ aryl, optionally substituted; and
a protecting group; and
z is 1, 2 or 3.

In some embodiments, z is 1. In some embodiments, z is 2. In some embodiments, z is 3.

In one embodiment, a compound of formula (V) is provided: 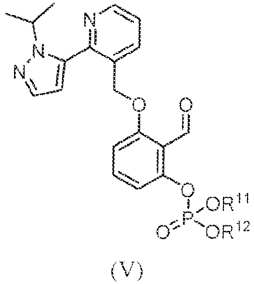 or a pharmaceutically acceptable salt thereof.

In some embodiments, R¹¹ and R¹² are independently C₁-C₆ alkyl. In some embodiments, R¹¹ and R¹² are selected from methyl, ethyl, or propyl. In some embodiments, R¹¹ and R¹² are methyl. In some embodiments, R¹¹ and R¹² are ethyl. In some embodiments, R¹¹ and R¹² are propyl. In some embodiments, R¹¹ and R¹² are C₁-C₆ alkyl substituted with 1-3 C₆-C₁₂ aryl groups, optionally substituted. In some embodiments, R¹¹ and R¹² are methyl substituted with 1-3 phenyl groups, optionally substituted. In some embodiments, R¹¹ and R¹² are methyl substituted with a phenyl group, optionally substituted with 1-3 groups selected from the group consisting of C₁-C₆ alkyl, C₁-C₆ alkoxy, halo and nitro.

In some embodiments, R¹¹ and R¹² are independently C₆-C₁₂ aryl, optionally substituted with 1-3 groups selected from the group consisting of C₁-C₆ alkyl, C₁-C₆ alkoxy, halo and nitro. In some embodiments, R¹¹ and R¹² are phenyl substituted with 1-3 groups selected from the group consisting of C₁-C₆ alkyl, C₁-C₆ alkoxy, halo and nitro. In some embodiments, R¹¹ and R¹² are phenyl.

In some embodiments, R¹¹ and R¹² independently are protecting groups. In some embodiments, the protecting groups are selected independently from the group consisting of 2-cyanoethyl, 2-cyano-1,1-dimethylethyl, 2-Benzamidoethyl, allyl, 4-methylthio-1-butyl, 2-(trimethylsilyl)ethyl, 2-(triphenylsilyl)ethyl, 2,2,2,-trichloroethyl, 4-methoxybenzyl, 4-nitrobenzyl, 2,4-dinitrobenzyl, 4-chlorobenzyl and fluorenyl-9-methyl.

In one aspect, provided herein is a compound of formula (V-A): 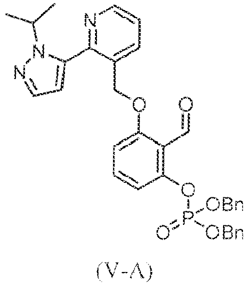 or a pharmaceutically acceptable salt thereof.

In one additional aspect, provided herein is a compound of formula (V-B) 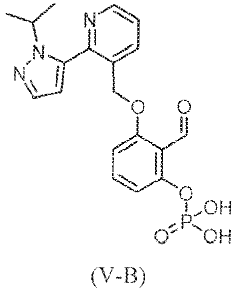 or a pharmaceutically acceptable salt thereof.

### Pharmaceutical Compositions

In another aspect, this invention provides a composition comprising any of the compounds described herein, and a pharmaceutically acceptable excipient.

Such compositions can be formulated for different routes of administration. Although compositions suitable for oral delivery will probably be used most frequently, other routes that may be used include transdermal, intravenous, intraarterial, pulmonary, rectal, nasal, vaginal, lingual, intramuscular, intraperitoneal, intracutaneous, intracranial, and subcutaneous routes. Suitable dosage forms for administering any of the compounds described herein include tablets, capsules, pills, powders, aerosols, suppositories, parenterals, and oral liquids, including suspensions, solutions and emulsions. Sustained release dosage forms may also be used, for example, in a transdermal patch form. All dosage forms may be prepared using methods that are standard in the art (see *e.g*., Remington's Pharmaceutical Sciences, 16th ed., A. Oslo editor, Easton Pa. 1980).

Pharmaceutically acceptable excipients are non-toxic, aid administration, and do not adversely affect the therapeutic benefit of the compound of this invention. Such excipients may be any solid, liquid, semi-solid or, in the case of an aerosol composition, gaseous excipient that is generally available to one of skill in the art. Pharmaceutical compositions in accordance with the invention are prepared by conventional means using methods known in the art.

The compositions disclosed herein may be used in conjunction with any of the vehicles and excipients commonly employed in pharmaceutical preparations, *e.g*., talc, gum arabic, lactose, starch, magnesium stearate, cocoa butter, aqueous or non-aqueous solvents, oils, paraffin derivatives, glycols, etc. Coloring and flavoring agents may also be added to preparations, particularly to those for oral administration. Solutions can be prepared using water or physiologically compatible organic solvents such as ethanol, 1,2-propylene glycol, polyglycols, dimethylsulfoxide, fatty alcohols, triglycerides, partial esters of glycerin and the like.

Solid pharmaceutical excipients include starch, cellulose, hydroxypropyl cellulose, talc, glucose, lactose, sucrose, gelatin, malt, rice, flour, chalk, silica gel, magnesium stearate, sodium stearate, glycerol monostearate, sodium chloride, dried skim milk and the like. Liquid and semisolid excipients may be selected from glycerol, propylene glycol, water, ethanol and various oils, including those of petroleum, animal, vegetable or synthetic origin, *e.g*., peanut oil, soybean oil, mineral oil, sesame oil, etc. In certain embodiments, the compositions provided herein comprises one or more of α-tocopherol, gum arabic, and/or hydroxypropyl cellulose.

In one embodiment, this invention provides sustained release formulations such as drug depots or patches comprising an effective amount of a compound provided herein. In another embodiment, the patch further comprises gum Arabic or hydroxypropyl cellulose separately or in combination, in the presence of alpha-tocopherol. Preferably, the hydroxypropyl cellulose has an average MW of from 10,000 to 100,000. In a more preferred embodiment, the hydroxypropyl cellulose has an average MW of from 5,000 to 50,000.

Compounds and pharmaceutical compositions of this invention maybe used alone or in combination with other compounds. When administered with another agent, the co-administration can be in any manner in which the pharmacological effects of both are manifest in the patient at the same time. Thus, co-administration does not require that a single pharmaceutical composition, the same dosage form, or even the same route of administration be used for administration of both the compound of this invention and the other agent or that the two agents be administered at precisely the same time. However, co-administration will be accomplished most conveniently by the same dosage form and the same route of administration, at substantially the same time. Obviously, such administration most advantageously proceeds by delivering both active ingredients simultaneously in a novel pharmaceutical composition in accordance with the present invention.

### Compounds and Pharmaceutical Compositions for use in Methods of Treatment

Described is a method for increasing tissue and/or cellular oxygenation, the method comprising administering to a subject in need thereof a therapeutically effective amount of any of the compounds or compositions described herein.

Described is a method for increasing oxygen affinity of hemoglobin S in a subject, the method comprising administering to a subject in need thereof a therapeutically effective amount of any of the compounds or compositions described herein.

In aspects of the invention, compounds or pharmaceutical compositions of the invention are provided for use in a method for treating a condition associated with oxygen deficiency.

In further aspects of the invention, compounds or pharmaceutical compositions of the invention are provided for use in a method for treating oxygen deficiency associated with sickle cell anemia.

In further aspects of the invention, compounds or pharmaceutical compositions of the invention are provided for use in a method for treating sickle cell disease. In still further aspects of the invention, compounds or pharmaceutical compositions of the invention are provided for use in a method for treating cancer, a pulmonary disorder, stroke, high altitude sickness, an ulcer, a pressure sore, Alzheimer's disease, acute respiratory disease syndrome, and a wound.

### Synthetic Methods

Described are certain methods for making the compounds described herein. The reactions are preferably carried out in a suitable inert solvent that will be apparent to the skilled artisan upon reading this disclosure, for a sufficient period of time to ensure substantial completion of the reaction as observed by thin layer chromatography, ¹H-NMR, etc. If needed to speed up the reaction, the reaction mixture can be heated, as is well known to the skilled artisan. The final and the intermediate compounds are purified, if necessary, by various art known methods such as crystallization, precipitation, column chromatography, and the likes, as will be apparent to the skilled artisan upon reading this disclosure.

An illustrative and non-limiting method for synthesizing a compound of formula (I), is schematically shown below.

Throughout the application, the following abbreviations have the following meanings. If not defined, the terms have their generally accepted meanings.
- °C: = degrees Celsius
- RT: = Room temperature
- min: = minute(s)
- h: = hour(s)
- µL: = Microliter
- mL: = Milliliter
- mmol: = Millimole
- eq: = Equivalent
- mg: = Milligram
- ppm: = Parts per million
- LC-MS: = Liquid chromatography-mass spectrometry
- HPLC: = High performance liquid chromatography
- NMR: = Nuclear magnetic resonance
- Ph₃PBr₂: = Triphenylphosphine dibromide
- DMF: = N, N-Dimethylformamide
- DCM: = Dichloromethane
- THF: = Tetrahydrofuran
- DIAD: = Diisopropyl azodicarboxylate
- DEAD: = Diethyl azodicarboxylate
- PEG: = Polyethylene glycol
- HβCD: = Hydroxy-propyl-β-cyclodextrin

In the following Schemes, "A" refers to substituent "A" as described herein. 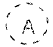 refers aryl or heteroaryl members of substituent "A" as described herein. 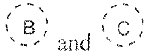 refer to rings B and C as described herein.

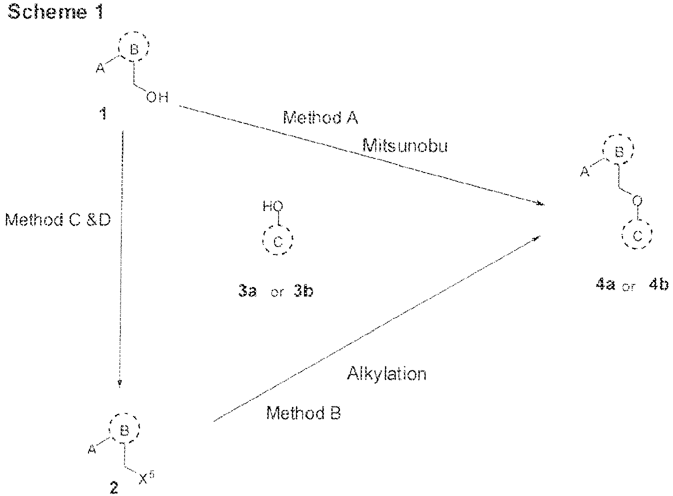

**General method A (Scheme 1) for preparing aryloxy/heteroarylether analogs (4a/4b) from substituted methylene alcohol (1) and hydroxyl (hetero)aryl aldehyde derivatives (3a/3b).** A hydroxyl (hetero)arylaldehyde derivatives (**3a/3b**) (0.1-2 mmol) mixture with substituted methylene alcohol (**1**) (0.8 to 1.2eq) and PPh₃ (1-1.5eq) in anhydrous THF (1-10mL) was stirred under nitrogen until complete dissolution. The solution was cooled to 0 °C on ice bath and DIAD or DEAD (1.1 cq) in THF or toluene was added dropwise over a 1-20 min period. The ice cooling bath was allowed to expire over 90 min and the mixture was stirred at RT for 2-48 hours. The mixture was stirred for 10 min, then filtered through a pad of silica. The silica was washed with ethyl acetate 2-20mL. The combined filtrates were evaporated and the residue was dried on highvac. The residue was purified by preparative HPLC or flash silica gel chromatography.

**General method A (Scheme 1) for preparing aryloxy/heteroarylether analogs (4a/4b) from substituted methylene halide (2) and hydroxyl (hetero)aryl aldehyde derivatives (3a/3b).** A mixture of hydroxyl (hetero)arylaldehyde derivatives (**3a/3b**) (0.1-2 mmol, 1-4 eq.), substituted methylene chloride or bromide (**2**) (1eq), and K₂CO₃ (2-5 eq.) (catalytic amount of NaI or Bu₄NI may also be added) in DMF or acetonitrile (1 to 10 mL) was stirred at RT or heating up to 120 °C for 0.5-8 h under nitrogen atmosphere. In workup A, water was added to the reaction mixture, the precipitated product was collected, washed with water, and then subjected to preparative HPLC or flash silica gel chromatography purification. In workup B (for products that did not precipitate), diluted HCl or aqueous NH₄Cl was added at 0 °C to adjusted the pH to ∼7, the reaction mixture was partitioned between ethyl acetate or dichloromethane and aqueous sodium chloride and the organic layer separated, dried, and solvent removed under vacuum to afford crude product which was purified by automated silica gel column chromatography using appropriate solvents mixture (*e.g.,* ethyl acetate/hexanes).

**General method C for preparing substituted methylene chloride (2a).** To a solution of substituted methylene alcohol (**1**) (0.1 to 2 mmol) in DCM (1-10 mL) was added SOCl₂ dropwise (2eq to 5eq) at 0 °C or RT. The reaction mixture was stirred at RT for 10 min to 6 h, or until reaction is judged complete (LC/MS). The reaction mixture is concentrated to dryness over a rotavap. The crude chloride residue was suspended in toluene, sonicated and concentrated to dryness. The process was repeated three times and dried under vacuum to give the substituted methylene chloride (**2**), usually as an off-white solid, which was used for next step without further purification. Alternatively, a solution of aqueous IN Na₂CO₃ is then added to produce a solution of pH∼ 8. the mixture was extracted with DCM (3 x 10-50mL), dried over sodium sulfate, and concentrated to the crude substituted methylene chloride (**2a**), which is then purified by column chromatography on silica gel (0-100% ethyl acetate-hexanes).

**General method D for preparing substituted methylene bromide (2b).** To a solution of substituted methylene alcohol (**1**) (0.1 to 2 mmol) in DCM (1-10 mL) was added Ph₃PBr₂ dropwise (2eq to 5eq) at 0 °C or RT. The reaction mixture was stirred at RT for 10 min to 2 h, or until reaction is judged complete (LC/MS). The reaction mixture is concentrated to dryness over a rotavap. The residue purified by column chromatography on silica gel (0-100% ethyl acetate-hexanes) to afford the pure bromide **2b.**

Syntheses of the ester prodrugs start with the free carboxylic acid bearing the tertiary amine. The free acid is activated for ester formation in an aprotic solvent and then reacted with a free alcohol group in the presence of an inert base, such as triethyl amine, to provide the ester prodrug. Activating conditions for the carboxylic acid include forming the acid chloride using oxalyl chloride or thionyl chloride in an aprotic solvent, optionally with a catalytic amount of dimethyl formamide, followed by evaporation. Examples of aprotic solvents, include, but are not limited to methylene chloride, tetrahydrofuran, and the like. Alternatively, activations can be performed in situ by using reagents such as BOP (benzotriazol-1-yloxytris(dimethylamino) phosphonium hexafluorolphosphate, and the like (see Nagy et al., 1993, Proc. Natl. Acad. Sci. USA 90:6373-6376) followed by reaction with the free alcohol. Isolation of the ester products can be affected by extraction with an organic solvent, such as ethyl acetate or methylene chloride, against a mildly acidic aqueous solution; followed by base treatment of the acidic aqueous phase so as to render it basic; followed by extraction with an organic solvent, for example ethyl acetate or methylene chroride; evaporation of the organic solvent layer; and recrystalization from a solvent, such as ethanol. Optionally, the solvent can be acidified with an acid, such as HCl or acetic acid to provide a pharmaceutically acceptable salt thereof. Alternatively the crude reaction can be passed over an ion exchange column bearing sulfonic acid groups in the protonated form, washed with deionized water, and eluted with aqueous ammonia; followed by evaporation.

Suitable free acids bearing the tertiary amine are commercially available, such as 2-(N-morpholino)-propionic acid, N,N- dimethyl-beta-alanine, and the like. Non- commercial acids can be synthesized in straightforward manner via standard literature procedures.

Carbonate and carbamate prodrugs can be prepared in an analogous way. For example, amino alcohols and diamines can be activated using activating agents such as phosgene or carbonyl diimidazole, to provide an activated carbonates, which in turn can react with the alcohol and/or the phenolic hydroxy group on the compounds utilized herein to provide carbonate and carbamate prodrugs.

Various protecting groups and synthetic methods related to them that can be used or adapted to make compounds of the invention can be adapted from the references Testa et al., Hydrolysis in Drug and Prodrug Metabolism, June 2003, Wiley- VCH, Zurich, 419-534 and Beaumont et al., Curr. Drug Metab. 2003, 4:461-85.

**Scheme 2** below provides a method of synthesizing an acyloxymethyl version of a prodrug by adapting a method from the reference Sobolev et al., 2002, J. Org. Chem. 67:401-410. 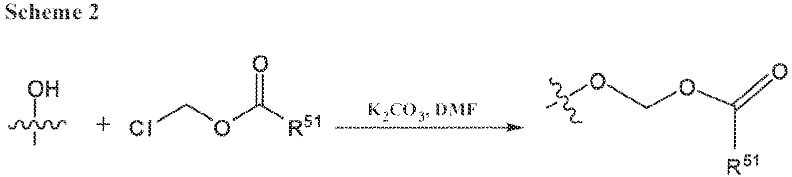 wherein R⁵¹ is C₁-C₆ alkyl.

**Scheme 3** below provides a method for synthesizing a phosphonooxymethyl version of a prodrug by adapting a method from Mantyla et al., 2004, J. Med. Chem. 47:188-195.

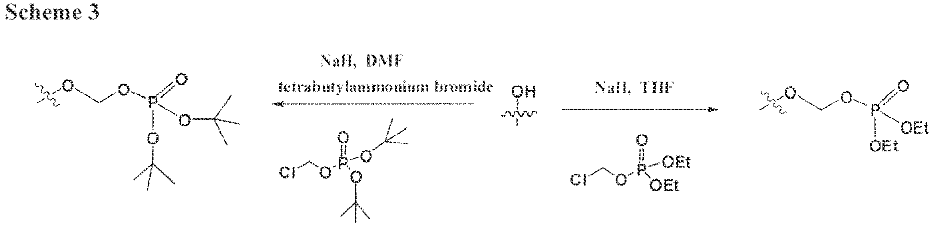

**Scheme 4** below provides a method of synthesizing an alkyloxymethyl version of a prodrug 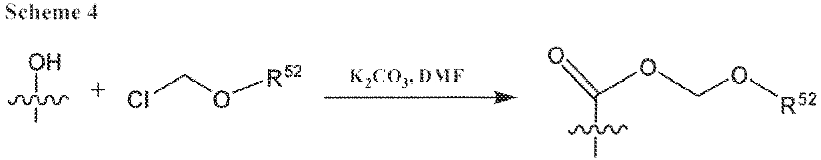 wherein R⁵² is C₁-C₆ alkyl, C₃-C₈ cycloalkyl, C₃-C₉ heterocyclyl, C₆-C₁₀ aryl, or C₃-C₉ heteroaryl.

The compound of formula (III) was synthesized as schematically described below and elaborated thereafter.

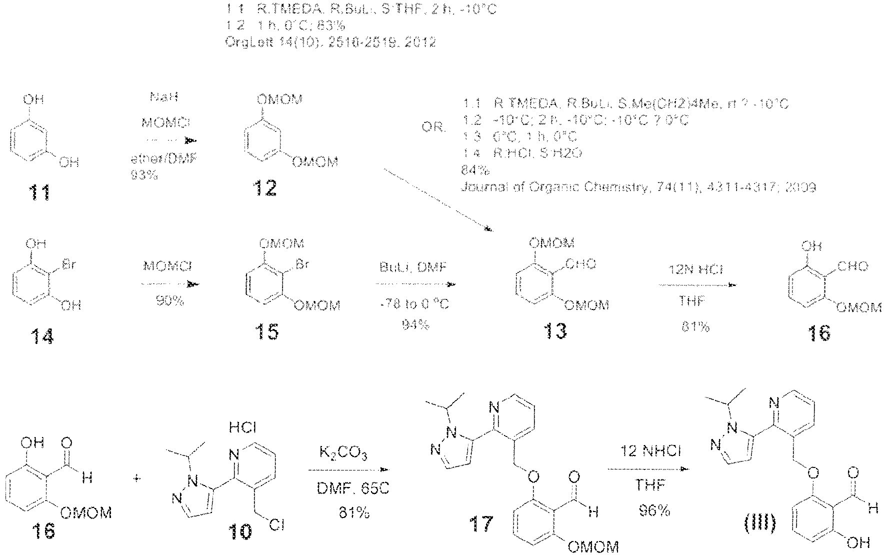

### Example 1: Synthesis of Compound 15

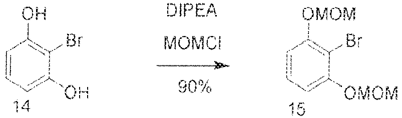

To a solution of 2-bromobenzene-1,3-diol (5 g, 26.45 mmol) in DCM (50 ml) at 0 °C was added DIPEA (11.54 mL, 66.13 mmol) and MOMCl (4.42 mL, 58.19 mmol). The mixture was stirred at 0 °C for 1.5 h, and then warmed to room temperature. The solution was diluted with DCM, washed with sat. NaHCO₃, brine, dried and concentrated to give crude product, which was purified by column (hexanes/EtOAc=4:1) to give desired product 15.58 g (90%).

### Example 2: Synthesis of Compound 13 from 15

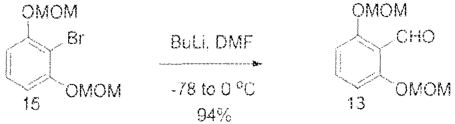

To a solution of 2-bromo-1,3-bis(methoxymethoxy)benzene (**15**) (19.9 g, 71.8 mmol) in THF (150 mL) at -78 °C was added BuLi (2.5 M, 31.6 mL, 79.0 mmol) dropwise. The solution was stirred at -78 °C for 25 min (resulting white cloudy mixture), then it was warmed to 0 °C and stirred for 25 min. The reaction mixture slowly turns homogenous. To the solution was added DMF at 0 °C. After 25 min, HPLC showed reaction completed. The mixture was quenched with sat. NH4Cl (150 mL), diluted with ether (300 mL). The organic layer was separated, aq. layer was further extracted with ether (2x200 mL), and organic layer was combined, washed with brine, dried and concentrated to give crude product, which was triturated to give 14.6 g desired product. The filtrate was then concentrated and purified by column to give additional 0.7 g, total mass is 15.3 g.

### Example 3: Synthesis of Compound 13 from resorcinol 11

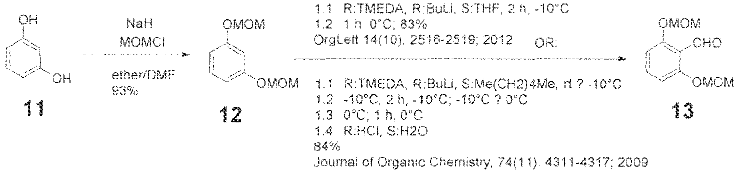

A three-necked round-bottom flask equipped with mechanical stirrer was charged with 0.22 mol of NaH (50 % suspension in mineral oil) under nitrogen atmosphere. NaH was washed with 2 portions (100 mL) of n-hexane and then with 300 mL of dry diethyl ether; then 80 mL of anhydrous DMF was added. Then 0.09 mol of resorcinol **11**, dissolved in 100 mL of diethyl ether was added dropwise and the mixture was left under stirring at rt for 30 min. Then 0.18 mol of MOMCl was slowly added. After 1 h under stirring at rt, 250 mL of water was added and the organic layer was extracted with diethyl ether. The extracts were washed with brine, dried (Na₂SO₄), then concentrated to give the crude product that was purified by silica gel chromatography to give compound **12** (93 % yield).

A three-necked round-bottom flask was charged with 110 mL of n-hexane, 0.79 mol of BuLi and 9.4 mL of tetramethylethylendiamine (TMEDA) under nitrogen atmosphere. The mixture was cooled at -10 °C and 0.079 mol of bis-phenyl ether **12** was slowly added. The resulting mixture was left under magnetic stirring at -10 °C for 2 h. Then the temperature was raised to 0 °C and 0.067 mol of DMF was added dropwise. After 1 h, aqueous HCl was added until the pH was acidic; the mixture was then extracted with ethyl ether. The combined extracts were washed with brine, dried (Na₂SO₄), and concentrated to give aldehyde **13** (84%).

2,6-bis(methoxymethoxy)benzaldehyde (13): mp 58-59 °C (n-hexane); IR (KBr) n: 1685 (C=O) cm⁻¹; ¹H-NMR (400 MHz. CDCl₃) δ 3.51 (s, 6H, 2 OCH₃), 5.28 (s, 4H, 2 OCH₂O), 6.84 (d, 2H, J = 8.40 Hz, H-3, H-5), 7.41 (t, 1H, J = 8.40 Hz, H-4), 10.55 (s, 1H, CHO); MS, m/e (relative intensity) 226 (M+, 3), 180 (4), 164 (14), 122 (2), 92 (2), 45 (100); Anal. Calc'd. for C₁₁H₁₄O₅: C,58.40; H, 6.24. Found: C, 57.98; H, 6.20.

### Example 4: The Synthesis of Compound 16

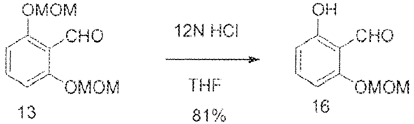

To a solution of 2,6-bis(methoxymethoxy)benzaldehyde (**13**) (15.3 g, 67.6 mmol) in THF (105 mL) (solvent was purged with N₂) was added conc. HCl (12N, 7 mL) under N₂, then it was further stirred under N₂ for 1.5 h. To the solution was added brine (100 mL) and ether (150 ml). The organic layer was separated and the aqueous layer was further extracted with ether (2x200 mL). The organic layer was combined, washed with brine, dried and concentrated to give crude product, which was purified by column (300 g, hexanes/EtOAc=85:15) to give desired product **16** (9.9 g) as yellow liquid.

### Example 5: Synthesis of Compound 17

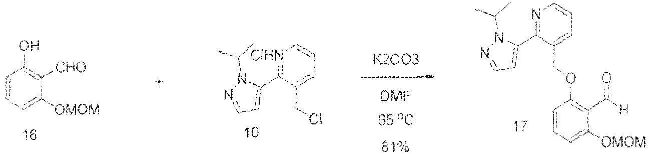

To a solution of 2-hydroxy-6-(methoxymethoxy)benzaldehyde (**16**) (10.88 g, 59.72 mmol) in DMF (120 mL) (DMF solution was purged with N₂ for 10 min) was added K₂CO₃ (32.05 g, 231.92 mmol) and 3-(chloromethyl)-2-(1-isopropyl-1H-pyrazol-5-yl)pyridine hydrochloride (**10**) (15.78 g, 57.98 mmol). The mixture was heated at 65 °C for 1.5 h, cooled to rt, poured into ice water (800 mL). The precipitated solids were isolated by filtration, dried and concentrated to give desired product (17, 18 g).

### Example 6: Synthesis of Compound (III)

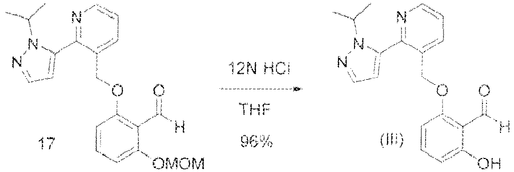

To a solution of 2-((2-(1-isopropyl-1H-pyrazol-5-yl)pyndin-3-yl)methoxy)-6-(methoxymethoxy)benzaldehyde **(17)** (18 g, 47.19 mmol) in THF (135 mL, solution was purged with N₂) was added conc. HCl (12N, 20 mL). The solution was stirred at rt for 3 h when HPLC showed the reaction complete. The mixture was added to a solution of NaHCO₃ (15 g) in water (1.2 L), and the resulting precipitate was collected by filtration, dried to give crude solid, which was further purified by column (DCM/EtOAc=60:40) to give pure product (15.3 g).

### Example 7: Synthesis of Compound III (free base) and its HCl salt form

Compound (III) free base (40 g) was obtained from the coupling of the alcohol intermediate **7** and 2,6-dihydroxybenzaldedhye **9** under Mitsunobu conditions. A procedure is also provided below:

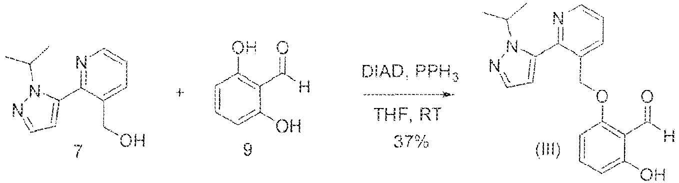

### Example 8: Synthesis of Compound (III) by Mitsunobu coupling

Into a 2000-mL three neck round-bottom flask, which was purged and maintained with an inert atmosphere of nitrogen, was placed a solution of [2-[1-(propan-2-yl)-1H-pyrazol-5-yl]pyridin-3-yl]methanol **(7)** (70 g, 322.18 mmol, 1.00 equiv) in tetrahydrofuran (1000 mL). 2,6-Dihydroxybenzaldehyde **(9)** (49.2 g, 356.21 mmol, 1.10 equiv) and PPh₃ (101 g, 385.07 mmol, 1.20 equiv) were added to the reaction mixture. This was followed by the addition of a solution of DIAD (78.1 g, 386.23 mmol. 1.20 equiv) in tetrahydrofuran (200 ml) dropwise with stirring. The resulting solution was stirred overnight at room temperature. The resulting solution was diluted with 500 ml of H₂O. The resulting solution was extracted with 3x500 ml of dichloromethane and the combined organic layers were dried over sodium sulfate and concentrated under vacuum. The residue was applied onto a silica gel column with EA:PE (1:50-1:3) as eluent to yield the crude product. The crude product was recrystallized from i-propanol/H₂O in the ratio of 1/1.5. This resulted in 40 g (37%) of 2-hydroxy-6-([2-[1-(propan-2-yl)-1H-pyrazol-5-yl]pyridin-3-yl]methoxy)benzaldehyde as a light yellow solid. The compound exhibited a melting point of 80-82 °C. MS (ES, *m*/*z*)*:* 338.1 [M+1]. ¹H NMR (300 MHz, DMSO-d6) δ 11.72(s, 1H), 10.21(s, 1H). 8.76(d. J=3.6Hz, 1H), 8.24(d, J=2.7Hz, 1H), 7.55(m, 3H), 6.55(m,3H) ,5.21 (s. 2H), 4.65 (m. 1H), 1.37 (d, J=5.1Hz, 6H), ¹H NMR (400 MHz, CDCl₃) δ 11.96 (s, 1H), 10.40 (s, 1H), 8.77 (dd, *J* = 4.8, 1.5 Hz, 1H), 8.00 (d, *J* = 7.8Hz, 1H), 7.63 (d, *J* = 1.8 Hz, 1H), 7.49 - 7.34 (m, 2H), 6.59 (d, *J* = 8.5 Hz, 1H), 6.37 (d, *J* = 1.8 Hz, 1H), 6.29 (d, *J* = 8.2 Hz, 1H), 5.10 (s, 2H), 4.67 (sep, *J* = 6.7 Hz, 1H), 1.50 (d, *J* = 6.6 Hz, 6H).

In another approach, multiple batches of Compound (III) free base are prepared in multi gram quantities (20g). The advantage of this route is the use of mono-protected 2,6-dihydroxybcnzaldchyde **(16),** which effectively eliminates the possibility of bis-alkylation side product. The mono-MOM ether of 2,6-dihydroxybenzatdehyde **(16)** can be obtained from two starting points, bromoresorcinol **(14)** or resorcinol **(11)** [procedures described in the Journal of Organic Chemistry, 74(11), 4311-4317; 2009]. All steps and procedures are provided below. Due to the presence of phenolic aldehyde group, precautions (i.e., carry out all reactions under inert gas such as nitrogen) should be taken to avoid oxidation of the phenol and/or aldehyde group.

### Example 9. Monophosphate Prodrug Formation

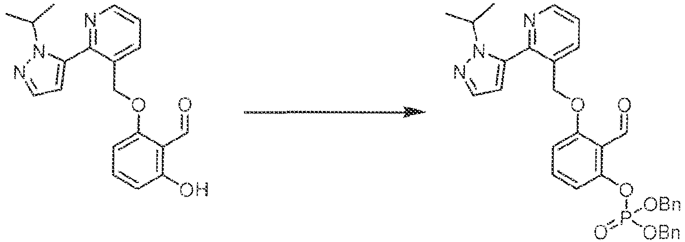

To a solution of 2-hydroxy-6-((2-(1-isopropyl-1H-pyrazol-5-yl)pyridin-3-yl)methoxy)benzaldehyde (1.06 g, 3.13 mmol)in dry acetonitrile was added N-ethyl-N-isopropylpropan-2-amine (1.10 mL, 6.27 mmol), N,N-dimethylpyridin-4-amine (0.038 g, 0.31 mmol) and carbon tetrachloride (1.52 mL, 15.7 mmol). The resulting mixture was purged with argon gas (15 minutes), cooled (-10 °C) and dibenzyl phosphonate (0.73 mL, 3.3 mmol) was added dropwise over 10 minutes. After 1 h the reaction mixture was diluted with sat'd KH₂PO₄ and extracted with EtOAc. The combined organic layers were washed with brine, dried over MgSO₄ and concentrated in vacuo. Purification by silica gel chromatography (10-100%-Et()Ac/hexanes) provided dibenzyl (2-formyl-3-((2-(1-isopropyl-1H-pyrazol-5-yl)pyridin-3-yl)methoxy)phenyl) phosphate (1.2 g, 65% yield). ¹H NMR (400 MHz, Chloroform-*d*) δ 10.39 (s, 1H), 8.72 (dd, *J* **=** 4.8, 1.7 Hz, 1H), 8.17 (ddt, *J* = 7.9, 1.5, 0.7 Hz, 1H), 7.61 (dd, *J* = 1.9, 0.5 Hz, 1H), 7.44 (dd, *J* = 7.9, 4.8 Hz, 1H), 7.37 (t*, J*= 8.4 Hz, 1H), 7.36 - 7.29 (m, 10H), 7.02 (dt, *J* = 8.4, 1.0 Hz, 1H), 6.66 (dt, *J* = 8.6, 0.8 Hz, 1H), 6.35 (d, *J* = 1.9 Hz, 1H), 5.19 (d, *J* = 1.5 Hz, 2H), 5.17 (d, *J*= 1.3 Hz, 2H), 5.06 (s, 2H), 4.66 4.58 (m, 1H), 1.47 (d, *J* = 6.6 Hz, 6H). ³¹P NMR (162 MHz, Chloroform-*d*) δ -7.09. MS (ES) for C₃₃H₃₂N₃O₆P: 598 (MH⁺).

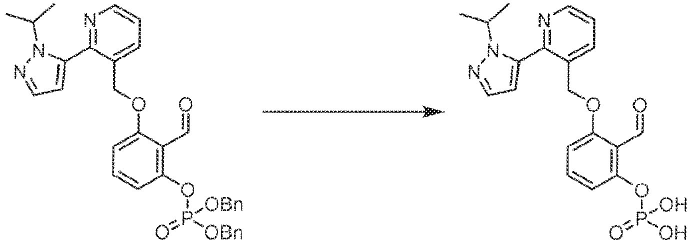

To a solution of (2-formyl-3-((2-(1-isopropyl-1H-pyrazol-5-yl)pyridin-3-yl)methoxy)phenyl) phosphate (0.600 g, 1.00 mmol) in MeOH (15 mL) was added Pd (10% on carbon, 50 mg). The reaction vessel was evacuated and then purged with an atmosphere of hydrogen three times. After 1 hour, the vessel was evacuated and purged with N₂ three times and filtered over Celite. The filter cake was washed with McOH and the combined filtrates were concentrated. The resulting residue was purified by preparatory HPLC to yield 2-formyl-3-((2-(1-isopropyl-1H-pyrazol-5-yl)pyridin-3-yl)methoxy)phenyl dihydrogen phosphate (32 mg, 8% yield). ¹H NMR (400 MHz, DMSO-d*₆*) δ 10.29 (s, 1H), 8.71 (dd, *J* = 4.7, 1.7 Hz, 1H), 8.27 (dd, *J* = 7.9, 1.7 Hz, 1H), 7.59 - 7.49 (m, 3H), 6.99 (d, *J* = 8.3 Hz, 1H), 6.86 (d, *J* = 8.5 Hz, 1H), 6.57 (d, *J* = 1.8 Hz, 1H), 5.11 (s, 2H), 4.63 (p, *J* = 6.6 Hz, 1H), 1.33 (d, *J* = 6.6 Hz, 7H). ³¹P NMR (162 MHz, DMSO-*d₆*) δ -6.46. MS (ES) for C₁₉H₂₀N₃O₆P: 418 (MH⁺).

### Example 10. Advantageous Pharmacokinetic Properties of the Monophosphate Prodrug

A sodium salt of the monophosphate prodrug compound (Formula (V-B)) was administered orally to rats in a dose of 10 mg/kg (5 mL/kg). The vehicle administered was dimefhylacetamide:PEG400:30%HpCD (5:25:70). The pharmacokinetic results are tabulated below and graphically illustrated in Figure 1.

**Blood**

| **Animal_#** | **T1/2 (hr)** | **Tmax (hr)** | **Cmax (ng/mL)** | **Cmax (uM)** | **AUCall (hr* ng/mL)** | **AUC(0-∞) (hr*ng/mL)** | **B/P Ratio** |
|---|---|---|---|---|---|---|---|
| 7 | 12.5 | 8 | 27500 | 81.5 | 464873 | 668159 | 50.9 |
| 8 | 24.0 | 4 | 29700 | 88.0 | 577713 | 1190229 | 67.1 |
| 9 | 19.2 | 8 | 21200 | 62.8 | 411205 | 740923 | 28.3 |
| Mean | 18.6 | 6.67 | 26133 | 77.5 | 484597 | 866437 | 48.7 |
| SD | 5.78 | 2.31 | 4412 | 13.1 | 84988 | 282762 | 19.5 |
| %CV | 31.1 | 34.6 | 16.9 | 16.9 | 17.5 | 32.6 | 39.9 |

**Plasma**

| **Animal_#** | **T1/2 (hr)** | **Tmax (hr)** | **Cmax (ng/mL)** | **Cmax (uM)** | **AUCall (hr*ng/mL)** | **AUC(0-∞) (hr*ng/mL)** |
|---|---|---|---|---|---|---|
| 7 | 19.3 | 0.5 | 1170 | 3.47 | 11315 | 17405 |
| 8 | 26.4 | 1 | 1380 | 4.09 | 11355 | 22007 |
| 9 | 28.1 | 1 | 1920 | 5.69 | 17414 | 36253 |
| Mean | 24.6 | 0.833 | 1490 | 4.42 | 13361 | 25222 |
| SD | 4.67 | 0.289 | 387 | 1.15 | 3510 | 9827 |
| %CV | 19.0 | 34.6 | 26.0 | 26.0 | 26.3 | 39.0 |

The monophospate prodrug was efficiently converted to compound of formula (III). At the monophosphate dose adminisered, the whole blood Cmax of the compound of formula (III) was about 78 uM which is equivalent to the Cmax achieved following administration of the compound of formula (III) at the same dose.

From the foregoing it will be appreciated that, although specific embodiments of the invention have been described herein for purposes of illustration, various modifications may be made without deviating from the scope of the invention.

## Claims

1. A compound of formula (II): 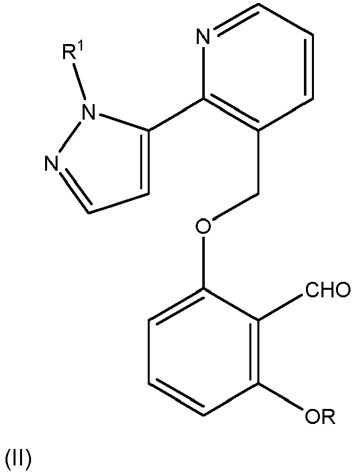 or an N oxide thereof, or a pharmaceutically acceptable salt of each thereof,
wherein R is a phosphate, a diphosphate, a phosphonate or a phosphoramidate containing moiety; or,
R is -C(O)R³¹, C(O)OR³¹, or CONR¹³R¹⁴, wherein each R³¹ is independently a C₁-C₆ alkyl, C₃-C₈ cycloalkyl, 4-9 membered heterocycle, or a 5-10 membered heteroaryl, containing at least 1 basic nitrogen moiety; and
R¹³ and R¹⁴ independently are C₁-C₆ alkyl, C₃-C₈ cycloalkyl, 4-9 membered heterocycle, or a 5-10 membered heteroaryl, containing at least 1 basic nitrogen moiety; or R¹³ and R¹⁴ together with the nitrogen atom they are bonded to form a 4-9 membered heterocycle substituted with at least 1 amino, C₁-C₆ alkyl amino, or di C₁-C₆ alkylamino group; or
R is C(O)(CH₂)ₘNR³⁴R³⁵ or C(O)O(CH₂)ₘNR³⁴R³⁵,
wherein m, R³⁴, and R³⁵ are defined as tabulated below:
| R | m | R³⁴ | R³⁵ | NR³⁴R³⁵ |
|---|---|---|---|---|
| C(O)(CH₂)ₘNR³⁴R³⁵ | 1 | Me | Me | |
| C(O)(CH₂)ₘNR³⁴R³⁵ | 2 | Me | Me | |
| C(O)(CH₂)ₘNR³⁴R³⁵ | 3 | Me | Me | |
| C(O)(CH₂)ₘNR³⁴R³⁵ | 4 | Me | Me | |
| C(O)(CH₂)ₘNR³⁴R³⁵ | 1 | | | 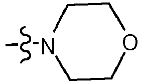 |
| C(O)(CH₂)ₘNR³⁴R³⁵ | 2 | | | 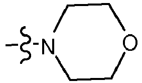 |
| C(O)(CH₂)ₘNR³⁴R³⁵ | 3 | | | 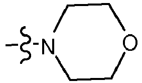 |
| C(O)(CH₂)ₘNR³⁴R³⁵ | 4 | | | 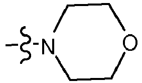 |
| C(O)O(CH₂)ₘNR³⁴R³⁵ | 2 | Me | Me | |
| C(O)O(CH₂)ₘNR³⁴R³⁵ | 3 | Me | Me | |
| C(O)O(CH₂)ₘNR³⁴R³⁵ | 4 | Me | Me | |
| C(O)O(CH₂)ₘNR³⁴R³⁵ | 2 | | | 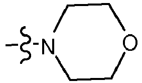 |
| C(O)O(CH₂)ₘNR³⁴R³⁵ | 3 | | | 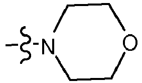 |
| C(O)O(CH₂)ₘNR³⁴R³⁵ | 4 | | | 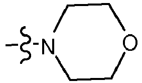 |
and R¹ is C₁-C₆ alkyl optionally substituted with 3-6 fluoro atoms.

2. The compound of claim 1, wherein R¹ is isopropyl.

3. The compound of claim 1 having formula (IV): 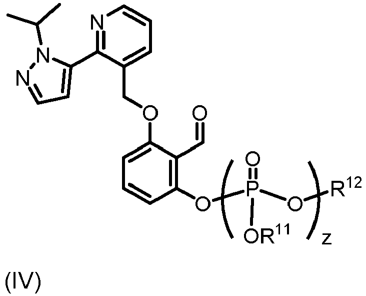 or a pharmaceutically acceptable salt thereof, wherein
R¹¹ and R¹² are independently selected from the group consisting of hydrogen;
C₁-C₆ alkyl optionally substituted with 1-3 C₆-C₁₂ aryl groups, optionally substituted; C₆-C₁₂ aryl, optionally substituted; and
a protecting group; and
z is 1, 2 or 3.

4. The compound of claim 3, wherein the compound is of formula (V-A): 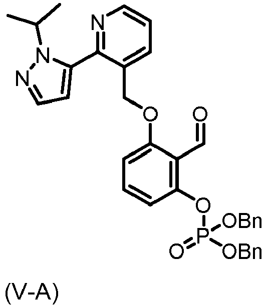 or a pharmaceutically acceptable salt thereof.

5. The compound of claim 3 of formula (V-B) 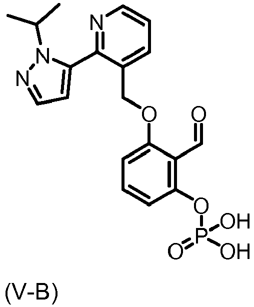 or a pharmaceutically acceptable salt thereof.

6. A pharmaceutical composition comprising a compound of any one of claims 1-5 and at least one pharmaceutically acceptable excipient.

7. A compound of any one of claims 1-5 or the pharmaceutical composition of claim 6 for use in a method for treating a condition associated with oxygen deficiency.

8. A compound of any one of claims 1-5 or the pharmaceutical composition of claim 6 for use in a method for treating cancer, a pulmonary disorder, stroke, high altitude sickness, an ulcer, a pressure sore, Alzheimer's disease, acute respiratory disease syndrome, or a wound.

9. A compound of any one of claims 1-5 or the pharmaceutical composition of claim 6 for use in a method for treating sickle cell disease.

## Patentansprüche

1. Verbindung mit der Formel (II): 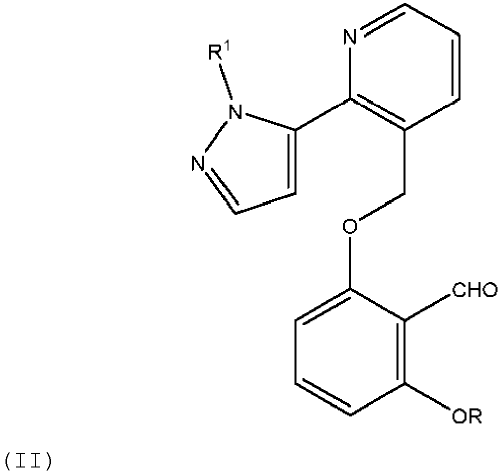 oder ein N-Oxid davon, oder ein pharmazeutisch annehmbares Salz von jedem davon,
wobei R ein phosphat-, diphosphat-, phosphonat- oder phosphoramidathaltiger Anteil ist; oder
R -C(O)R³¹, -C(O)OR³¹ oder -CONR¹³R¹⁴ ist, wobei jedes R³¹ unabhängig ein C₁-C₆-Alkyl, C₃-C₈-Cycloalkyl, 4-9-gliedriger Heterocyclus oder ein 5-10-gliedriges Heteroaryl ist, das mindestens 1 basischen Stickstoffanteil enthält; und
R¹³ und R¹⁴ unabhängig C₁-C₆-Alkyl, C₃-C₈-Cycloalkyl, 4-9-gliedriger Heterocyclus oder ein 5-10-gliedriges Heteroaryl sind, das mindestens 1 basischen Stickstoffanteil enthält; oder R¹³ und R¹⁴ zusammen mit dem Stickstoffatom, an das sie gebunden sind, einen 4-9-gliedrigen Heterocyclus bilden, der mit mindestens 1 Amino-, C₁-C₆-Alkylamino- oder Di-C₁-C₆-alkylaminogruppe substituiert ist; oder
RC(O)(CH₂)ₘNR³⁴R³⁵ oder C(O)O(CH₂)ₘNR³⁴R³⁵ ist, wobei m, R³⁴ und R³⁵ wie in der folgenden Tabelle wiedergegeben definiert sind:
| R | m | R³⁴ | R³⁵ | NR³⁴R³⁵ |
|---|---|---|---|---|
| C(O)(CH₂)ₘNR³⁴R³⁵ | 1 | Me | Me | |
| C(O)(CH₂)ₘNR³⁴R³⁵ | 2 | Me | Me | |
| C(O)(CH₂)ₘNR³⁴R³⁵ | 3 | Me | Me | |
| C(O)(CH₂)ₘNR³⁴R³⁵ | 4 | Me | Me | |
| | | | | |
| C(O)(CH₂)ₘNR³⁴R³⁵ | 1 | | | 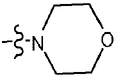 |
| C(O)(CH₂)ₘNR³⁴R³⁵ | 2 | | | 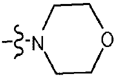 |
| C(O)(CH₂)ₘNR³⁴R3⁵ | 3 | | | 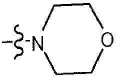 |
| C(O)(CH₂)ₘNR³⁴R³⁵ | 4 | | | 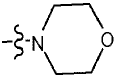 |
| C(O)O(CH₂)ₘNR³⁴R³⁵ | 2 | Me | Me | |
| C(O)O(CH₂)ₘNR³⁴R³⁵ | 3 | Me | Me | |
| C(O)O(CH₂)ₘNR³⁴R³⁵ | 4 | Me | Me | |
| C(O)O(CH₂)ₘNR³⁴R³⁵ | 2 | | | 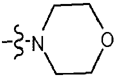 |
| C(0)0(CH₂)ₘNR³⁴R³⁵ | 3 | | | 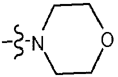 |
| C(O)O(CH₂)ₘNR³⁴R³⁵ | 4 | | | 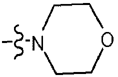 |
und R¹ C₁-C₆-Alkyl ist, das gegebenenfalls mit 3-6 Fluoratomen substituiert ist.

2. Verbindung nach Anspruch 1, wobei R¹ Isopropyl ist.

3. Verbindung nach Anspruch 1 mit der Formel (IV): 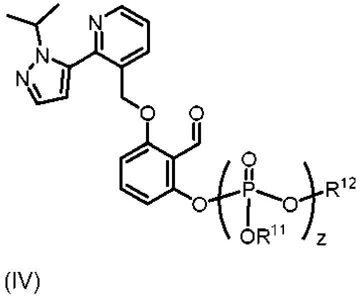 oder ein pharmazeutisch annehmbares Salz davon, wobei:
R¹¹ und R¹² unabhängig ausgewählt ist aus der Gruppe bestehend aus
Wasserstoff;
C₁-C₆-Alkyl, das gegebenenfalls mit 1-3 C₆-C₁₂-Arylgruppen substituiert ist, die gegebenenfalls substituiert sind;
C₆-C₁₂-Aryl, das gegebenenfalls substituiert ist; und
einer Schutzgruppe; und
z 1, 2 oder 3 ist.

4. Verbindung gemäß Anspruch 3, worin die Verbindung die Formel (V-A) aufweist: 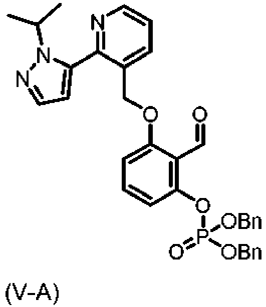 oder ein pharmazeutisch verträgliches Salz davon.

5. Verbindung nach Anspruch 3 mit der Formel (V-B): 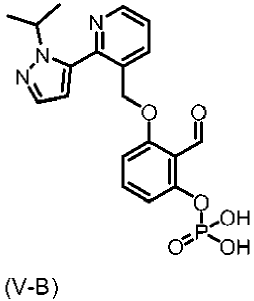 oder ein pharmazeutisch verträgliches Salz davon.

6. Pharmazeutische Zusammensetzung, umfassend eine Verbindung gemäß einem der Ansprüche 1-5 und mindestens einen pharmazeutisch akzeptablen Hilfsstoff.

7. Verbindung nach einem der Ansprüche 1-5 oder pharmazeutische Zusammensetzung nach Anspruch 6 zur Verwendung in einem Verfahren zur Behandlung eines Zustands, der mit Sauerstoffmangel zusammenhängt.

8. Verbindung nach einem der Ansprüche 1-5 oder pharmazeutische Zusammensetzung nach Anspruch 6 zur Verwendung in einem Verfahren zur Behandlung von Krebs, einer Lungenerkrankung, Schlaganfall, Höhenkrankheit, einem Ulcus, einem Druckgeschwür, Morbus Alzheimer, akutem Atemwegerkrankungssyndrom oder einer Wunde.

9. Verbindung nach einem der Ansprüche 1-5 oder pharmazeutische Zusammensetzung nach Anspruch 6 zur Verwendung in einem Verfahren zur Behandlung von Sichelzellenanämie.

## Revendications

1. Composé de formule (II) : 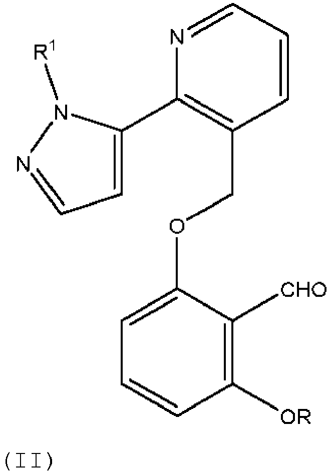 ou N oxyde de celui-ci, ou sel pharmaceutiquement acceptable de chacun de ceux-ci,
dans lequel R est un fragment contenant un phosphate, un diphosphate, un phosphonate ou un phosphoramidate ; ou, R est -C(O)R³¹, C(O)OR³¹, ou CONR¹³R¹⁴, dans lequel
chaque R³¹ est indépendamment un alkyle en C₁-C₆, un cycloalkyle en C₃-C₈, un hétérocycle de 4 à 9 chaînons, ou un hétéroaryle de 5 à 10 chaînons, contenant au moins 1 fragment d'azote basique ; et
R¹³ et R¹⁴ sont indépendamment un alkyle en C₁-C₆, un cycloalkyle en C₃-C₈, un hétérocycle de 4 à 9 chaînons, ou un hétéroaryle de 5 à 10 chaînons, contenant au moins 1 fragment d'azote basique ; ou R¹³ et R¹⁴, conjointement avec l'atome d'azote auquel ils sont liés, forment un hétérocycle de 4 à 9 chaînons substitué par au moins 1 groupe amino, (alkyle en C₁-C₆)amino, ou di(alkyle en C₁-C₆) amino ; ou
R est C(O) (CH₂)ₘNR³⁴R³⁵ ou C(O)O(CH₂)ₘNR³⁴R³⁵, dans lesquels m, R³⁴, et R³⁵ sont tels que définis dans le tableau ci-dessous :
| R | m | R³⁴ | R³⁵ | NR³⁴R³⁵ |
|---|---|---|---|---|
| C(O)(CH₂)ₘNR³⁴R³⁵ | 1 | Me | Me | |
| C(O)(CH₂)ₘNR³⁴R³⁵ | 2 | Me | Me | |
| C(O)(CH₂)ₘNR³⁴R³⁵ | 3 | Me | Me | |
| C(O)(CH₂)ₘNR³⁴R³⁵ | 4 | Me | Me | |
| | | | | |
| C(O)(CH₂)ₘNR³⁴R³⁵ | 1 | | | 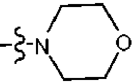 |
| C(O)(CH₂)ₘNR³⁴R³⁵ | 2 | | | 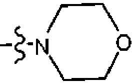 |
| C(O)(CH₂)ₘNR³⁴R³⁵ | 3 | | | 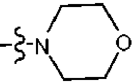 |
| C(O)(CH₂)ₘNR³⁴R³⁵ | 4 | | | 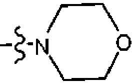 |
| C(O)O(CH₂)ₘNR³⁴R³⁵ | 2 | Me | Me | |
| C(O)O(CH₂)ₘNR³⁴R³⁵ | 3 | Me | Me | |
| C(O )O(CH₂)ₘNR³⁴R³⁵ | 4 | Me | Me | |
| C(O)O(CH₂)ₘNR³⁴R³⁵ | 2 | | | 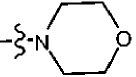 |
| C(O)O(CH₂)ₘNR³⁴R³⁵ | 3 | | | 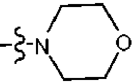 |
| C(O)O(CH₂)ₘNR³⁴R³⁵ | 4 | | | 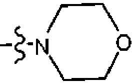 |
et R¹ est alkyle en C₁-C₆ facultativement substitué par 3 à 6 atomes de fluor.

2. Composé selon la revendication 1, dans lequel R¹ est isopropyle.

3. Composé selon la revendication 1 ayant la formule (IV) : 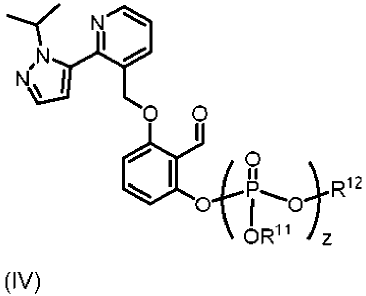 ou sel pharmaceutiquement acceptable de celui-ci, dans lequel
R¹¹ et R¹² sont indépendamment choisis dans le groupe constitué de
hydrogène ;
alkyle en C₁-C₆ facultativement substitué par 1 à 3 groupes aryle en C₆-C₁₂, facultativement substitués ; aryle en C₆-C₁₂, facultativement substitué ; et
un groupe protecteur ; et
z est 1, 2 ou 3.

4. Composé selon la revendication 3, le composé étant de formule (V-A) : 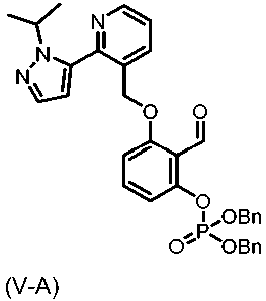 ou sel pharmaceutiquement acceptable de celui-ci.

5. Composé selon la revendication 3 de formule (V-B) 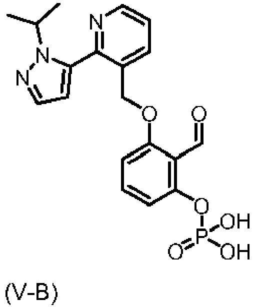 ou sel pharmaceutiquement acceptable de celui-ci.

6. Composition pharmaceutique comprenant un composé selon l'une quelconque des revendications 1 à 5 et au moins un excipient pharmaceutiquement acceptable.

7. Composé selon l'une quelconque des revendications 1 à 5 ou composition pharmaceutique selon la revendication 6, pour utilisation dans un procédé pour traiter une affection associée à un déficit en oxygène.

8. Composé selon l'une quelconque des revendications 1 à 5 ou composition pharmaceutique selon la revendication 6 pour utilisation dans un procédé de traitement d'un cancer, un trouble pulmonaire, un accident vasculaire cérébral, le mal aigu des montagnes, un ulcère, une escarre de décubitus, la maladie d'Alzheimer, le syndrome de détresse respiratoire aigu ou une plaie.

9. Composé selon l'une quelconque des revendications 1 à 5 ou composition pharmaceutique selon la revendication 6 pour utilisation dans un procédé pour traiter la drépanocytose.
